# EUROPÄISCHE PATENTSCHRIFT

(11) **EP 0 708 841 B1**
(45) Veröffentlichungstag und Bekanntmachung des Hinweises auf die Patenterteilung: **10.11.1999**
(21) Anmeldenummer: 94924756.3
(22) Anmeldetag: 13.07.1994
(51) Int. Cl.: C12Q 1/68, C12N 15/11, C07K 14/47, C07K 16/18, C07K 16/30, G01N 33/577

(54) **VERFAHREN ZUR IDENTIFIZIERUNG VON MENSCHLICHEN UND TIERISCHEN ZELLEN MIT DER FÄHIGKEIT ZU UNBEGRENZTER PROLIFERATION ODER ZUR TUMORBILDUNG**
METHOD OF IDENTIFYING HUMAN AND ANIMAL CELLS CAPABLE OF UNLIMITED PROLIFERATION OR TUMOUR FORMATION
PROCEDE D'IDENTIFICATION DE CELLULES HUMAINES ET ANIMALES SUSCEPTIBLES DE PROLIFERER DE MANIERE ILLIMITEE OU DE GENERER DES TUMEURS

(30) Priorität: 15.07.1993 DE 4323727
(43) Veröffentlichungstag der Anmeldung: 01.05.1996
(73) Patentinhaber: Roche Diagnostics GmbH, 68298 Mannheim (DE)
(72) Erfinder: ABKEN, Hinrich Johann, D-56414 Meudt-Dahlen (DE); ALBERT, Winfried, D-82390 Eberfing (DE); JUNGFER, Herbert, D-82319 Starnberg (DE)
(86) Internationale Anmeldenummer: EP9402307
(87) Internationale Veröffentlichungsnummer: WO9502701

(56) Entgegenhaltungen:
- EP-A- 0 576 862
- ANNALS OF THE NEW YORK ACADEMY OF SCIENCE, Bd.684, 10. Juni 1993, NY ACADEMY OF SCIENCE, NY,US; Seiten 193 - 195 H. ABKEN ET AL. 'A DNA-binding zinc-protein increases the immortalizing activity of an extrachromosomal DNA sequence from mouse L929 cells'
- DATABASE WPI Week 8613, Derwent Publications Ltd., London, GB; AN 86-115284 & JP,A,61 033 199 (ASAHI CHEMICAL IND KK) 17. Februar 1986
- PROC. NATL. ACAD SCI., Bd.85, Nr.2, Januar 1988, NATL. ACAD SCI., WASHINGTON, DC, US; Seiten 468 - 472 H. ABKEN ET AL. 'Immortalization of human lymphocytes by transfection with DNA from mouse L929 cytoplasts' in der Anmeldung erwähnt
- PROC. NATL. ACAD SCI., Bd.90, Nr.14, 15. Juli 1993, NATL. ACAD SCI., WASHINGTON, DC, US; Seiten 6518 - 6522 H. ABKEN ET AL. 'Short DNA sequences from the cytoplasm of mouse tumor cells induce immortalization of human lymphocytes in vitro'
- PROC. NATL. ACAD. SCI. USA RASSOULZADEGAN ET AL.: 'Expression of the large T protein of polyoma virus promotes the establishment in culture of "normal" rodent fibroblast cell lines' Bd. 80, Nr. 14, Juli 1983, USA, Seiten 4354 - 4358
- ONCOGENE STRAUSS ET AL.: 'Inmortalization and transformation of human fibroblasts by regulated expression of polyoma virus T antigens' Bd. 5, Nr. 8, August 1990, Seiten 1223 - 1229
- CARCINOGENESIS MEDINA UND KITTREL: 'Inmortalization phenotype dissociated from the preneoplastic phenotype in mouse mammary epithelial outgrowths in vivo' Bd. 14, Nr. 1, 1993, Seiten 25 - 28
- CANCER CELLS STRAUSS UND GRIFFIN: 'Cellular Immortalization - An Essential Step or Merely a Risk Factor in DNA Virus-induced Transformation?' Bd. 2, 1990, Seiten 360 - 365
- ONCOGENE FREUND ET AL.: 'Separation of inmortalization from tumor induction with polyoma large T mutants that fail to bind the retinoblastoma gene product' Bd. 7, 1992, Seiten 1979 - 1987
- PROC. NATL. ACAD. SCI. USA O'BRIEN ET AL.: 'Suppression of tumor growth by senescence in virally transformed human fibroblasts' Bd. 83, November 1986, USA, Seiten 8659 - 8663
- J. EXP. MED. BUSCHLE ET AL.: 'Interferon gamma inhibits apoptopic cell death in B cell chronic lymphocytic leukemia' Bd. 177, Nr. 1, 01 Januar 1993, Seiten 213 - 218

## Beschreibung

Die Erfindung betrifft DNS-Protein-Komplexe, Proteine, DNS-Sequenzen und Antikörper, welche zum Nachweis von Zellen mit der Fähigkeit zu unbegrenzter Proliferation oder zur Tumorbildung geeignet sind, Verfahren zur Gewinnung von solchen DNS-Protein-Komplexen, Proteinen oder DNS-Sequenzen sowie deren Verwendung zur Identifizierung von tierischen und menschlichen Zellen mit der Fähigkeit zu unbegrenzter Proliferation oder zur Tumorbildung.

Alle differenzierten menschlichen und tierischen Zellen haben ein begrenztes Potential zur Zellvermehrung (Proliferation) in vivo und in vitro, ehe sie der Zellalterung (Seneszenz) und dem Zelltod unterliegen. Die Anzahl der zu einem bestimmten Zeitpunkt noch möglichen Zellteilungen einer Zelle ist vom Differenzierungsgrad der Zelle, vom Alter und der Spezies des Spenders, von dem die Zelle abstammt, sowie von der Dauer der gegebenenfalls bereits begonnenen Kultivierung der Zellen abhängig (Goldstein, S.: Replicative Senescence, Science 249 (1990), 1129 - 1133). Eine unbegrenzte Proliferation findet sich dagegen häufig bei neoplastisch transformierten Zellen. Sie bilden dadurch stetig wachsende Zellverbände, die zur Ausbildung eines Tumors und schließlich zusammen mit der veränderten Ausprägung anderer Eigenschaften dieser Zellen zur Tumorerkrankung führen. Klinisch zeichnen sich derartige maligne Tumore gegenüber gutartigen Tumoren durch ihr rasches Wachstum und häufige Metastasenbildung aus. Die neoplastische Transformation der Zelle ist gekennzeichnet durch ein heterogenes Bild zahlreicher Änderungen der zellulären Morphologie und Physiologie, die auch vom Differenzierungsgrad der Zellen abhängig sind. Bislang sind nur wenige molekular definierbare Parameter der neoplastischen Transformation bekannt, wie z. B. ein veränderter Methylierungsgrad bestimmter Gene (W. Doerfler et al., Eukaryotic DNA methylation: facts and problems, FEBS Letters 268 (1990), 329 - 330), eine veränderte Genexpression oder eine veränderte Phosphorylierung bestimmter Genprodukte.

Gemeinsam ist den Tumorzellen in der überwiegenden Mehrzahl dagegen die Fähigkeit zur unbegrenzten Proliferation in vivo. Aufgrund verschiedener Beobachtungen wird angenommen, daß die von Wachstumsfaktoren unabhängige Proliferation der Tumorzellen zwar eine häufig vorhandene, aber nicht unbedingt notwendige Eigenschaft tumorbildender Zellen darstellt (M. Strauss, B.E. Griffin, Cellular Immortalization, Cancer Cells 2 (1990), 360 - 365). Für zahlreiche Fragestellungen der Diagnostik und Therapie ist es aber dennoch von entscheidender Bedeutung, diejenigen Zellen aufzufinden, die der normalen Proliferationskontrolle nicht mehr unterliegen und somit das Potential zur unbegrenzten Proliferation haben.

Dabei ergibt sich die Schwierigkeit, diese bei Tumorzellen gefundene Fähigkeit zu unbegrenzter Proliferation zu unterscheiden von der in vielen Geweben gefundenen Fähigkeit zur Regeneration. Diese stellt eine kontrollierte, kurzzeitige (transiente) Zellvermehrung und eine nur vorübergehende Unterdrückung des programmierten Zelltods als Antwort auf eine physiologische Stimulation dar. Die transiente Proliferation normaler Zellen wird nach Regeneration des Gewebes durch noch nicht identifizierte Signale wieder gehemmt.

Die bekannten Verfahren zum Nachweis von malignen Tumorzellen beruhen auf klinischen, histologischen und zytologischen Beobachtungen sowie veränderten physiologischen Meßwerten. Diagnostische Routine-Verfahren erfolgen zumeist aufgrund histologischer Untersuchungen am Gewebeschnitt (W.A.D. Anderson und J.M. Kissane, Pathology I, II, Mosby, Saint Louis 1977, 7th edition; C.S. Herrington und J.O'D. McGee, Diagnostic Molecular Pathology, Oxford University Press, Vol. I, II, 1st ed., 1992). Der Verband maligner Zellen ist im Gegensatz zu gutartigen Wucherungen meist nur unscharf zum Nachbargewebe abgegrenzt und die Zellen wachsen infiltrierend und destruierend in das umgebende Gewebe ein. Meist liegt zusätzlich eine perifokale Entzündung vor. Häufig weisen eine große Anzahl von Mitosen auf die gesteigerte Proliferationsaktivität der Tumorzellen hin.

Neben diesen histologischen Untersuchungen werden zum Nachweis proliferierender maligner Zellen Antikörper eingesetzt, welche Antigene erkennen, die bevorzugt von proliferierenden Tumorzellen exprimiert werden, wie z. B. Ki67 (D.C. Matthews, F.O. Smith, I.D. Bernstein, Monoclonal antibodies in the study and therapy of hematopoietic cancers, Curr. Opinion Immunol. 4 (1992), 641 - 646; M. Schwouzen, V. Diehl, M. Pfreundschuh, Immunozytologische Phänotypisierung von Leukämien und Lymphomen, Med. Klinik 85 (1990), 533 - 547). Diese Verfahren beruhen jedoch auf der Bestimmung indirekter Parameter, ohne die molekularen Prozesse, die im Zusammenhang mit der unbegrenzten Proliferation stehen, nachzuweisen.

Aufgabe der Erfindung ist es, Zellen mit dem Potential zu unbegrenzter Proliferation, insbesondere maligne Tumorzellen schnell und sicher zu identifizieren, ohne daß dafür diese Zellen in vitro kultiviert oder nach Injektion in Versuchstieren propagiert werden müssen. Dabei sollen Zellen mit dem Potential zu unbegrenzter Proliferation von Zellen mit transienter Proliferation in einem regenerierenden Gewebeverband unterschieden werden können.

Diese Aufgabe wird gelöst durch einen DNS-Protein-Komplex (im weiteren als Komplex bezeichnet), welcher zum Nachweis von menschlichen oder tierischen Zellen mit der Fähigkeit zu unbegrenzter Proliferation oder zur Tumorbildung geeignet ist und welcher erhältlich ist durch Isolierung einer Mitochondrien-freien Fraktion des Zytoplasmas aus permanent teilungsfähigen menschlichen oder tierischen Zellen, die in einem Cäsiumchlorid-Gradienten eine Dichte von etwa 1,82 - 1,89 g/cm³ aufweist, und Isolierung des Komplexes aus dieser Fraktion durch Extraktion mit Phenol und Fällung mit Ethanol.

Es hat sich überraschenderweise gezeigt, daß Zellen mit der Fähigkeit zu unbegrenzter Proliferation oder zur Tumorbildung im Gegensatz zu normalen ruhenden Zellen, seneszenten Zellen oder Zellen mit transienter Proliferation derartige Komplexe im Zytoplasma aufweisen. Über diese erfindungsgemäßen Komplexe können dann solche Zellen nachgewiesen werden, welche z. B. infolge einer malignen Entartung ein unbegrenztes Wachstumspotential aufweisen. Dazu wird Zytoplasma oder eine geeignete Zytoplasmafraktion mit einem erfindungsgemäßen Komplex als Standard in einer Gelelektrophorese, durch Bestimmung des DNA Gehalts oder Reaktion mit spezifischen Antikörpern verwendet.

In Annals ofthe New York Academy of Science, Band 684, Seiten 193 - 195, ist beschrieben, daß eine DNA namens LC 108 mit einer Länge von 203 bp kontinuierliche in vitro Proliferation von Lymphozyten induzieren kann. Es ist ferner angesprochen, daß diese DNA mit cytoplasmatischen Proteinen und Zn²⁺ Ionen DNA Protein-Komplexe bilden kann.

In Derwent Abstract AN86-115284 und JP-A-61033199 ist die Sequenz für ein humanes β-Interferon-Gen beschrieben.

In Proceedings ofthe National Academy of Science USA, Vol. 90, Seiten 6518 - 6522 vom 15.07.1993 sind die DNA-Sequenzen von LC108 und EFC38 aus dem Cytoplasma von Maus-Tumor-Zellen beschrieben, die auf menschliche Lymphozyten in vitro eine immortalisierende Wirkung haben.

Bei EP-A-0 576 862 handelt es sich um ein Dokument, welches zwischen dem Prioritätstag der vorliegenden Anmeldung und dem Anmeldetag der internationalen Patentanmeldung publiziert wurde. Es beschreibt die Sequenz von LC108 DNA und EFC38 DNA deren Verwendung zur Immortalisierung sowie spezielle Oligonukleotidstartermoleküle.

Zur Isolierung der erfindungsgemäßen Komplexe werden Zytoplasten von permanent teilungsfähigen menschlichen oder tierischen Zellen nach bekannten Verfahren gewonnen (s. z. B. EP-B-0 093 436, EP-B-0 256 512 und Proc. Natl. Acad. Sci. 85 (1988), 468 - 472) und lysiert, z. B. mit Hilfe von Detergenzien wie NP40 oder SDS (Wigler und Weistein, Biochem. Biophys. Res. Comm. 63 (1975) 669 - 674). Die Mitochondrien werden aus dieser Zytoplastenfraktion abgetrennt, vorzugsweise mit Hilfe eines Sucrosegradienten (J. Biol. Chem. 249 (1974) 7991 - 7995). Diejenigen Fraktionen, die keine Mitochondrien enthalten, werden mit RNase, vorzugsweise RNase A und RNase Tl sowie mit Proteinase wie z. B. Proteinase K und Pronase inkubiert. Aus diesem Ansatz wird eine Fraktion, welche in einem Cäsiumchloridgradienten eine Dichte von ca. 1,82 - 1,89 g/cm³ aufweist, angereichert und aus dieser Fraktion ein Komplex durch Extraktion mit Phenol und Fällung mit Ethanol isoliert. Die Gewinnung einer Fraktion, welche in einem Cäsiumchloridgradienten eine Dichte von ca. 1,82-1,89 g/cm³ aufweist, kann dabei sowohl mit Hilfe einer Zentrifugation in einem CsCl-Dichtegradienten als auch über eine elektrophoretische Auftrennung nach dem Fachmann geläufigen Verfahren erfolgen (Sambrook et al., Molecular Cloning, Cold Spring Harbor Laboratory, 2nd edition, 1989).

Die zur Isolierung der Komplexe verwendete Mitochondrienfreie Fraktion des Zytoplasmas kann alternativ auch durch wiederholtes Einfrieren und Auftauen der Zellen oder durch Gewinnung einer Zellfraktion, welche frei von nukleärer DNS ist, erhalten werden. Eine solche Fraktion gewinnt man bevorzugt durch Lyse der Zellen mit Natriumchlorid und SDS ("Hirt-Extraktion", J. Mol. Biol. 26 (1967) 365 - 369) und anschließender Zentrifugation. Aus dem Überstand können dann die Komplexe isoliert werden.

In einem bevorzugten Verfahren erfolgt die Isolierung der Komplexe über eine Sedimentation aus einem zytoplasmatischen Lysat der zu untersuchenden Zellen mit Hilfe eines Salz-Gradienten. Die Zellen werden dazu in einem Mg²⁺-haltigen Puffer (50 mmol/l Tris-HCl, pH 7,2, 10 mmol/l EDTA, 3 mmol/l MgCl₂) durch mehrmaliges Einfrieren und Auftauen ohne Verwendung von Detergentien lysiert, die Zellkerne durch Zentrifugation bei 6000 x g sedimentiert und der Überstand mit Proteinase K (100 µg/ml) 1 h bei 60°C verdaut. Die freigesetzten Komplexe werden durch Zentrifugation (150 000 x g, 10 h) durch einen CsC1-Zwei-Stufen-Gradienten isoliert, wobei die untere Fraktion die Dichte von ca. 1.80 g/cm³ hat, die obere Fraktion eine Dichte von ca. 1.70 g/cm³. Dabei sedimentieren die zytoplasmatischen Komplexe, während die chromosomale DNS oder Komplexe ohne kovalente DNS-Protein-Bindungen im Überstand verbleiben.

Dieses Verfahren erlaubt eine sehr schnelle Isolierung zytoplasmatischer Komplexe, wobei folgende Eigenschaften ausgenutzt werden:
- Die Komplexe werden durch das Einfrieren und Auftauen im Mg²⁺-haltigen Puffer freigesetzt, ohne daß die Zellkerne lysieren oder Detergentien verwendet werden müssen.
- Die Komplexe sind stabil gegenüber Proteinasen, während zytoplasmatische Organellen und Membranen abgebaut werden.
- Die Komplexe haben eine sehr hohe Dichte (ca. 1,86 g/cm³) und können so mit Hilfe von Salz-Gradienten von chromosomaler oder anderer zytoplasmatischer DNS abgetrennt werden.
- Die DNS-Protein-Bindung ist stabil gegenüber hohen Salzkonzentrationen.

Die Komplexe können aus dem Zytoplasma von beliebigen transformierten Zellen, insbesondere permanent wachsenden menschlichen oder tierischen Zellen oder von Tumorzellen, insbesondere von solchen, die aus Tumorbiopsien erhalten wurden, gewonnen werden. Unter transformierten Zellen sind in diesem Zusammenhang Zellen zu verstehen, die durch ein Agens unsterblich gemacht wurden. Permanent proliferierende Zellen verschiedener Spezies (z. B. Mensch, Maus, Ratte) und verschiedenen Differenzierungsgrades (Fibrom-, Myelom-, Asziteszellen) enthalten Komplexe, die sich in ihrem Protein- und/oder DNS-Anteil unterscheiden. Die erfindungsgemäßen Komplexe sind also für Spezies und Differenzierungsgrad der Zellen spezifisch. Vorzugsweise werden daher für den Nachweis von Zellen mit Fähigkeit zu unbegrenzter Proliferation oder Fähigkeit zur Tumorbildung Komplexe aus Zellen der gleichen Spezies und eines ähnlichen Zelltyps wie der zu untersuchenden Zelle isoliert. So sind z. B. zum Nachweis von Maus-Fibroblasten/Fibromzellen bzw. Maus-Aszites-Tumorzellen mit unbegrenzter Proliferation Komplexe besonders bevorzugt, die aus Zytoplasten transformierter Maus-L-Zellen (L929-Zellen, ATCC CCL 1) bzw. Ehrlich-Aszites-Zellen (ATCC CCL 77) nach dem beschriebenen Verfahren isoliert wurden. Zur Gewinnung von Komplexen von permanent proliferierenden Zellen anderer Gewebe oder Differenzierungsgrade sowie Zellen anderer Spezies können für das beschriebene Verfahren andere, dem Fachmann geläufige Tumorzellinien der gewünschten Differenzierungsgrade und Spezies als Quelle zur Isolierung der Komplexe verwendet werden. So werden z. B. zur Gewinnung entsprechender Komplexe aus humanen Cervix-Tumoren vorzugsweise die humanen Zellinien HeLa oder aus B-Lymphom-Linien die B-Zell-Lymphomlinie BJAB verwendet. Weiterhin können dabei auch permanent wachsende Zellinien verwendet werden, die aufgrund einer experimentellen Immortalisierung z. B. durch Fusion primärer Zellen mit Zytoplasten permanent wachsender Zellen (EP-B-0 093 436, Abken et al., J. Cell Biol. 130 (1986) 795 - 805) oder durch Transfektion mit DNS aus diesen Zytoplasten (EP-B-0 256 512 und DE 42 18 945.4 und Abken et al., Proc. Natl. Acad. Sci. 85 (1988) 468 - 472) immortalisiert wurden.

Es hat sich gezeigt, daß in Zellen des Hodgkin-Lymphoms eine zytoplasmatische DNS-Fraktion der Dichte 1,86 g/cm³ persistiert. Diese DNS ist ebenfalls mit Proteinen verknüpft. Die in der Fraktion enthaltenen DNS-Moleküle haben eine Länge zwischen 50 und 500 bp. Wie bei Zellen aus Maus-Tumoren handelt es sich um lineare DNS-Moleküle. Sie hybridisieren jedoch nicht mit denen aus Maus-Tumor-Zellen. Dies zeigt, daß sowohl tierische als auch menschliche Lymphomzellen erfindungsgemäße zytoplasmatische DNS-Sequenzen enthalten. Die erfindungsgemäße Fraktion findet sich auch in Mensch-Tumor-Zellen des Colon- und des Mammakarzinoms sowie des menschlichen Melanoms.

Aus den Komplexen können die Proteine bzw. die DNA isoliert werden, über die dann ebenfalls ein Nachweis von menschlichen oder tierischen Zellen mit unbegrenzter Proliferation oder der Fähigkeit zur Tumorbildung ermöglicht wird.

Ein weiterer Gegenstand der Erfindung sind daher Proteine mit einem Molekulargewicht von ca. 52, 62 bzw. 64 kD, welche zum Nachweis von menschlichen oder tierischen Zellen mit der Fähigkeit zu unbegrenzter Proliferation oder zur Tumorbildung geeignet sind und welche erhältlich sind durch Behandlung eines erfindungsgemäßen Komplexes mit DNase I und chromatographischer oder elektrophoretischer Isolierung der dabei freigesetzten ca. 52, 62 bzw. 64 kD großen Proteine.

Zur Isolierung dieser Proteine werden die Komplexe mit DNase I behandelt und die abgebaute Nukleinsäure durch Gelfiltration von den Proteinen abgetrennt, die dabei zugleich nach der Größe aufgetrennt werden. Unter Verwendung von Komplexen aus Maus-L-Zellen (L929) bzw. Ehrlich Aszites-Tumorzellen werden dabei Proteine mit einer Größe von 52, 62 und 64 kD erhalten. Diese Proteine sind charakterisiert dadurch, daß sie die erfindungsgemäß klonierte pLC 108 DNS (SEQ ID NO 1) in Gegenwart von 8 mM Zn²⁺ zu binden vermögen. Die Proteine aus Ehrlich-Aszites-Zellen sind dadurch gekennzeichnet, daß sie die klonierte pEFC38 DNS (SEQ ID NO 29) in Gegenwart von 8 mM Na⁺ zu binden vermögen. Vorzugsweise wird für die Präparation dieser Proteine eine matrixgebundene DNase eingesetzt, z. B. eine kovalent an Sepharose gekoppelte DNase.

Ein weiterer Gegenstand der Erfindung sind Antikörper, welche zum Nachweis von menschlichen oder tierischen Zellen mit der Fähigkeit zu unbegrenzter Proliferation oder zur Tumorbildung geeignet sind und welche erhältlich sind durch Immunisierung von Tieren mit einem erfindungsgemäßen Komplex oder Protein oder DNS. Hierbei können alle üblicherweise zur Immunisierung verwendeten Tiere, wie Mäuse (z. B. Stamm MNRI oder BALB/c) oder Kaninchen, und Immunisierungsprotokolle verwendet werden (siehe z. B. J. Peters und H. Baumgarten, Monoklonale Antikörper, Springer Verlag, 2. Auflage 1988).

Bei Immunisierung mit den erfindungsgemäßen Komplexen, DNS oder Proteinen werden Antikörper erhalten, welche spezifisch an Proteine aus dem Zytoplasma von Zellen mit der Fähigkeit zu unbegrenzter Proliferation oder zur Tumorbildung binden, nicht jedoch an Proteine aus Zellen mit normaler Proliferationskapazität. Der Nachweis dieser Proteine mit Hilfe der erfindungsgemäßen Antikörper kann dabei nach den dem Fachmann geläufigen Immunoassaymethoden wie z. B. ELISA, Fluoreszenz-, Immunoassay, kompetitiver Immunoassay erfolgen.

Neben dem Proteinanteil der erfindungsgemäßen Komplexe eignet sich für den Nachweis auch der DNS-Anteil.

Ein weiterer Gegenstand der Erfindung ist daher eine DNS, welche zum Nachweis von menschlichen oder tierischen Zellen mit der Fähigkeit zu unbegrenzter Proliferation oder zur Tumorbildung geeignet ist und welche erhältlich ist durch Klonierung oder enzymatische Vermehrung der DNS eines erfindungsgemäßen Komplexes.

Es hat sich überraschenderweise gezeigt, daß die DNS des erfindungsgemäßen Komplexes wie eine isolierte DNS in Klonierungsvektoren ligiert werden kann oder enzymatisch z. B. in einer PCR amplifiziert werden kann, obwohl diese DNS in den Komplexen so fest an die Proteine gebunden ist, daß sie weder durch Detergenzien, Proteasen, hohe Salzkonzentrationen noch durch Erhitzen ablösbar ist. Die DNS kann gewonnen werden, indem die DNS der Komplexe z. B. in geläufige Vektoren, wie z. B. pUC19, ligiert und in dem Fachmann geläufigen Wirtsorganismen, wie z. B. E. coli HB101 oder E. coli DH5a, kloniert wird. Die rekombinanten Klone, die die erfindungsgemäße DNS-Sequenz enthalten, werden dann durch Hybridisierung mit den erfindungsgemäßen Komplexen identifiziert und die DNS isoliert. Auf diese Weise konnten 20 rekombinante Plasmidklone aus L929-Zellen (ATCC CCL 1) sowie 25 rekombinante Plasmidklone aus Ehrlich-Aszites-Zellen (ATCC CCL 77) erhalten werden, welche zur molekularen Identifizierung von Zellen mit der Fähigkeit zu unbegrenzter Proliferation geeignet sind. Diese DNS-Sequenzen sind in den Sequenzprotokollen SEQ ID NO 1 - 45 aufgeführt. Mit Hilfe dieser Sequenzen ist es möglich, weitere geeignete DNS-Sequenzen anderer Zellen verschiedener Differenzierungsgrade oder anderer Spezies zur Identifizierung von entsprechenden Zellen mit Fähigkeit zu unbegrenzter Proliferation aufzufinden.

Die in SEQ ID NO 1 und SEQ ID NO 29 gezeigten DNS-Sequenzen sind zur Immortalisierung von humanen oder tierischen Zellen geeignet (vergleiche auch Deutsche Patentanmeldung P 42 18 945.4). Die weiteren DNS-Sequenzen (SEQ ID NO 2 - 28 und SEQ ID NO 30 - 45) haben keine immortalisierende Wirkung, sind aber zum Nachweis von menschlichen oder tierischen Zellen mit der Fähigkeit zu unbegrenzter Proliferation oder zur Tumorbildung geeignet.

Ein bevorzugter Gegenstand der Erfindung ist daher eine erfindungsgemäße DNS, welche eine der in SEQ ID NO 2 - 28 oder SEQ ID NO 30 - 45 gezeigten DNS-Sequenzen enthält oder mit einer dieser Sequenzen hybridisiert.

Ein weiterer bevorzugter Gegenstand der Erfindung sind DNS, welche eine der in SEQ ID NO 46-61 gezeigten DNS-Sequenzen enthält oder mit einer oder mehrerer dieser Sequenzen hybridisiert. Die Nukleinsäuren SEQ ID NO 46-58 sind aus DNA von Hodgkin-Zellen der Linie HD 428 abgeleitet. Die Sequenzen der SEQ ID NO 59-61 sind aus DNA-Klonen von MCF 7-Zellen (Mamma-Karzinom) abgeleitet.

Die erfindungsgemäßen Nukleinsäuren können DNS, jedoch auch RNS oder Nukleinsäureanaloge, z.B. solche bei denen das Zuckerphosphatgerüst durch eine Polypeptidkette ersetzt ist, sein. Die Nukleinsäure kann doppelsträngig sein, bevorzugt ist sie jedoch einzelsträngig. Die Erfindung bezieht sich außerdem auf die jeweils komplementären Sequenzen. Bevorzugt enthalten die erfindungsgemäßen Nukleinsäuren eine Sequenz von mindestens 15 Aminosäuren, wobei diese Sequenz von mindesten 15 Aminosäuren ausgewählt ist aus
- einer Sequenz die in einer Sequenz nach SEQ ID NO 2-28 oder 30-61 enthalten ist,
- einer Sequenz, die sich durch Aneinanderhängen zweier Sequenzen der SEQ ID NO 1-45, 46-58 oder 59-61 ergibt,
- Sequenzen, die für dieselbe Aminosäuresequenz codieren, wie die beiden oben genannten Typen,
- Sequenzen, die zu den beiden oben genannten Typen mindestens zu 70 %, bevorzugt mehr als 90 %, besonders bevorzugt mehr als 95 % innerhalb der spezifischen Sequenzen aufweisen.

Die Nukleinsäuren sind besonders bevorzugt zwischen 16 und 100 Nukleotiden lang. Je nach Länge können sie nach molekularbiologischen oder chemisch/synthetischen Methoden hergestellt werden.

Überraschenderweise hat sich erwiesen, daß die meisten der bisher untersuchten DNS-Sequenzen die Konsenussequenz NNAAANTNTNGAANTGTANNANTGNAA (SEQ ID NO 62) aufweisen.

Ein weiterer Gegenstand der Erfindung ist ein Verfahren zur Gewinnung einer erfindungsgemäßen DNS durch Isolierung eines erfindungsgemäßen Komplexes aus permanent teilungsfähigen menschlichen oder tierischen Zellen und Klonierung oder enzymatische Vermehrung der DNS aus diesem Komplex.

Ein weiterer bevorzugter Gegenstand der Erfindung ist ein Verfahren zur Gewinnung einer erfindungsgemäßen DNS, bei welchem die erfindungsgemäßen DNS-Sequenzen durch Hybridisierung einer genomischen Genbank oder eine cDNS-Bank von menschlichen oder tierischen Zellen mit einer der in SEQ ID NO 1 - 61 gezeigten DNS-Sequenzen identifiziert und die hybridisierende klonierte DNS aus der Genbank nach bekannten Verfahren isoliert wird. Hierzu können Genbanken sowohl aus permanent proliferierenden Tumorzellen als auch aus normalen Zellen oder Geweben verwendet werden.

Ein ebenfalls bevorzugter Gegenstand der Erfindung ist ein Verfahren zur Gewinnung einer erfindungsgemäßen DNS durch Bindung der DNS von menschlichen oder tierischen Zellen an ein erfindungsgemäßes Protein, wobei die Proteine auf Nitrozellulose oder andere Trägermembranen gebunden werden und in Gegenwart von Zn²⁺ oder Na⁺ (z. B. 4°C für 30 Minuten) inkubiert werden. Anschließend wird stringent bei hoher Salzkonzentration (z. B. 0,1 % SDS, 2 x SSC) gewaschen.

Ein besonders bevorzugter Gegenstand der Erfindung ist ein Verfahren zur Gewinnung einer erfindungsgemäßen DNS durch enzymatische Vermehrung einer DNS- oder RNS-Fraktion von menschlichen oder tierischen Zellen, wobei OligonukleotidStartermoleküle verwendet werden, die mit einer der in SEQ ID NO 1 - 61 gezeigten Sequenzen hybridisieren. Vorzugsweise werden dabei Oligonukleotid-Startermoleküle verwendet, welche eine Länge von 20 - 30 Basenpaaren aufweisen. Die enzymatische Vermehrung der gewünschten DNS-Sequenzen mit Hilfe der Polymerasekettenreaktion (PCR) erfolgt gemäß dem Fachmann bekannten Verfahren (Mullis und Fallona, Meth. Enzymol. 155 (1987) 335 - 350, Saiki et al., Science 239 (1988) 487 - 491). Dabei können als DNS-Fraktionen sowohl Genbanken als auch DNS- oder RNS-Präparationen oder Zellysate von permanent proliferierenden oder normalen seneszenten Zellen des Menschen oder von Tieren verwendet werden.

Eine andere Variante des erfindungsgemäßen Verfahrens ist schließlich die chemische Synthese einer erfindungsgemäßen DNS, welche eine der in SEQ ID NO 1 - 61 gezeigten DNS-Sequenzen oder Teile dieser DNS-Sequenzen aufweist. Diese Synthese erfolgt gemäß den üblichen dem Fachmann geläufigen Oligonukleotidsyntheseverfahren (F. Eckstein, Hrsg., Oligonucleotides and analogures: A practical approach, IRL Press at Oxford University Press, Oxford (1991)), vorzugsweise mit Hilfe handelsüblicher Oligonukleotidsyntheseapparaturen.

Ein weiterer Gegenstand der Erfindung ist ein Verfahren zum Nachweis von menschlichen oder tierischen Zellen mit Fähigkeit zu unbegrenzter Proliferation oder zur Tumorbildung durch Nachweis eines erfindungsgemäßen Komplexes in einer Mitochondrien-freien Fraktion des Zytoplasmas der zu untersuchenden Zelle. Normale Zellen mit begrenzter Proliferationskapazität enthalten keine derartigen Komplexe in dieser Fraktion des Zytoplasmas, während Zellen mit unbegrenzter Proliferation, wie z. B. Tumorzellen, mehrere hundert Kopien dieser Komplexe aufweisen.

Die erfindungsgemäßen Komplexe werden sezerniert. Die DNS-Protein-Verknüpfung dieser Komplexe ist sehr stabil gegenüber proteolytischem Abbau. Die Komplexe sind mehrere Wochen lang in Zellüberständen nachweisbar.

Die Komplexe können auch im peripheren Blut, im Urin oder anderen Körperflüssigkeiten enthalten sein und können dort nachgewiesen werden.

Ein bevorzugter Gegenstand der Erfindung ist daher ein Verfahren zum Nachweis von menschlichen oder tierischen Zellen mit unbegrenztem Proliferationspotential oder Tumorbildung durch Nachweis eines erfindungsgemäßen Komplexes in Körperflüssigkeiten.

Die Komplexe können über ihren DNS-Gehalt oder gelelektrophoretisch bestimmt werden, wobei erfindungsgemäße Komplexe als Standard für die Ermittlung einer positiven Reaktion mitgeführt werden können. Bei diesem Verfahren kann jedoch nicht zwischen Komplexen aus verschiedenen Spezies oder Gewebetypen unterschieden werden. Ein solcher spezifischer Nachweis ist jedoch unter Verwendung erfindungsgemäßer Antikörper möglich.

Neben kompletten Antikörpern können auch Antikörperfragmente verwendet werden, die die spezifischen Antigen-Erkennungsregionen der Leichten- oder Schweren-Kette (V_{L} bzw. V_{H}) des Antikörpers beinhalten. Diese Fragmente können mit Trägern, wie z. B. Proteinen, Zuckern oder Sepharose, oder mit Farbstoffen zu Fusionsprodukten gekoppelt werden.

Ein solches Fusionsprodukt kann vorzugsweise dadurch erhalten werden, daß man die kodierenden Gen-Sequenzen für die Antigen-Erkennungsregionen V_{L} und V_{H} des Antikörpers mit den Gensequenzen für ein Trägermolekül fusioniert, z. B. ein Oberflächen-Protein eines Phagen oder ein CapsidProtein eines Virus, und dieses Hybrid-Gen dann in vitro exprimiert. Die Verfahren zur Isolierung und Klonierung der für die V-Regionen des Antikörpers kodierenden Gensequenzen sowie deren Insertion in die DNS-Sequenz anderer Gene, z. B. in die Gensequenz für das Phagenprotein g3p, sowie Methoden zur Expression eines solchen Fusionsproteins sind dem Fachmann bekannt (Orlandi et al., Proc. Natl. Acad. Sci. USA 86 (1989): 3833 - 3837; Chiang et al., BioTechniques 7 (1989): 360 - 366; Winter, G., und Milstein, C., Nature 349 (1991): 293 - 299). Die Fusionsprodukte (auf der Oberfläche von Phagen oder Viren oder in isolierter Form) können dann wie ein Antikörper zum Nachweis der Komplexe eingesetzt werden.

Schließlich können die Komplexe in den zu untersuchenden Zellen auch durch Hybridisierung einer DNS-Fraktion dieser Zellen mit einer erfindungsgemäßen DNS nachgewiesen werden. Der Vorteil dieses Verfahrens ist, daß mit derartigen DNS-Proben zwischen Zellen verschiedener Differenzierungsgrade (Fibrom- bzw. Azites-Tumor) und Spezies (Maus bzw. Mensch) unterschieden werden kann.

Ein weiterer Gegenstand der Erfindung ist daher ein Verfahren zum Nachweis von menschlichen oder tierischen Zellen mit der Fähigkeit zu unbegrenzter Proliferation oder Tumorbildung durch Hybridisierung einer DNS-Fraktion dieser Zellen mit einer erfindungsgemäßen DNS.

Als DNS-Fraktion kann dabei eine cytoplasmatische Zellfraktion verwendet werden, welche erfindungsgemäße Komplexe enthält. Es ist jedoch auch möglich, hierfür die nukläre, chromosomale DNS der zu untersuchenden Zellen einzusetzen. Es hat sich nämlich gezeigt, daß nukläre, chromosomale DNS von normalen transient proliferierenden Zellen im Vergleich zu Zellen mit der Fähigkeit zu unbegrenzter Proliferation nach Restriktionsverdau und Analyse der erhaltenen Restriktionsfragmente in einem Southern-Blot, ein anderes Muster an hybridisierenden Restriktionsfragmenten bei Hybridisierung einer erfindungsgemäßen DNS-Sequenz ergeben. In der Regel zeigen zusätzliche mit den Proben hybridisierende Restriktionsfragmente das Vorhandensein von Zellen mit der Fähigkeit zu unbegrenzter Proliferation oder zur Tumorbildung an.

Die erfindungsgemäßen DNS-Sequenzen weisen eine so hohe Sensitivität und Spezifität auf, daß diese Proben auch zur Hybridisierung mit Zellen oder Gewebe in situ eingesetzt werden können. Dabei werden nur diejenigen Zellen im Cytoplasma markiert, die eine Fähigkeit zu unbegrenzter Proliferation oder zur Tumorbildung zeigen. Für diese in situ-Hybridisierung können sowohl Zellen verwendet werden, die in vitro kultiviert wurden, als auch Zellen aus Biopsien oder Gewebeschnitten von Biopsien, ohne daß ein Kultivierungsschritt der Zellen zur Vermehrung an Zellmaterial erforderlich ist.

Ein bevorzugter Gegenstand der Erfindung ist daher eine Variante des erfindungsgemäßen Verfahren, bei welcher die Hybridisierung als in situ-Hybridisierung durchgeführt wird.

Die Empfindlichkeit des Verfahrens kann noch weiter gesteigert werden, indem die DNS der Komplexe aus den zu untersuchenden Zellen enzymatisch, insbesondere mit Hilfe der Polymerasekettenreaktion gemäß der dem Fachmann bekannten Methode (Mullis and Fallona, Meth. Enzymol. 155 (1987): 335 - 350; Saiki et al., Science 239 (1988), 487 - 491) für die eigentliche Nachweisreaktion vermehrt werden.

Ein bevorzugter Gegenstand der Erfindung ist daher eine besondere Ausführungsform des erfindungsgemäßen Verfahrens, bei welcher aus einem Lysat der zu untersuchenden Zellen oder einer DNS- oder RNS-Fraktion dieser Zellen eine Nukleinsäure enzymatisch vermehrt wird, unter Verwendung von Oligonukleotiden, die mit einer erfindungsgemäßen DNS hybridisieren.

Eine solche Amplifikation ist möglich, obwohl die DNS-Proteinbindung stabil gegenüber hohen Temperaturen (1 Stunde bei 90°C), hohen Salzkonzentrationen (wie z. B. 6,9 mol/l Cäsiumchlorid) und gegenüber einem Proteinase K-Verdau (100 µg/ml, 1 Stunde bei 60°C) ist.

Die beschriebenen Verfahren zum Nachweis von Zellen mit der Fähigkeit zu unbegrenzter Proliferation oder zur Tumorbildung finden Anwendung zum Nachweis maligner Tumoren, wobei die Zellen einer Biopsie, eines Gewebeschnitts oder Zellen oder sezernierte Komplexe aus Körperflüssigkeiten von Menschen oder Tieren als Probenmaterial eingesetzt werden. Die beschriebenen Verfahren eignen sich somit auch zum Auffinden solcher Zellen. Weiterhin können die beschriebenen Verfahren in entsprechend abgewandelter Form auch zur Isolierung derartiger Zellen verwendet werden. Hierbei dienen die beschriebenen Verfahren insbesondere zur Identifizierung der gewünschten Zellen in einer bestimmten Fraktion während des Isolierungsverfahrens. Dazu können erfindungsgemäße Antikörper eingesetzt und die Zellen anschließend durch Fluoreszenz-aktiviertes Sortieren (FACS) isoliert werden.

Die Erfindung wird durch die folgenden Beispiele in Verbindung mit den Sequenzprotokollen, welche bevorzugte erfindungsgemäße DNS-Sequenzen zeigen, näher erläutert.

### Beispiel 1

### Isolierung von Komplexen und Klonierung der DNS dieser Komplexe, welche zum Nachweis von Zellen mit der Fähigkeit zu unbegrenzter Proliferation oder Tumorbildung geeignet sind

Zytoplasmatische Komplexe werden aus Maus-Tumor-Fibroblasten der Linie L929 (L929-Zellen, ATCC CCL 1) sowie aus Ehrlich-Aszites-Zellen (EAZ, ATCC CCL 77) isoliert. Zunächst werden Zytoplasten dieser Zellen nach bekannten Verfahren (Wigler und Weistein, Biochem. Biophys. Res. Comm. 63 (1975) 669 - 674) unter Verwendung von Cytochalasin B (50 µg/ml) gewonnen und durch wiederholtes Einfrieren und Auftauen lysiert. Die Mitochondrien werden aus dem Zytoplasten-Lysat mit Hilfe eines Sucrosegradienten gemäß J. Biol. Chem. 249 (1974) 7991 - 7995 abgetrennt. Die Fraktionen, die keine Mitochondrien enthalten, werden 30 Minuten bei 37°C mit RNase A (20 µg/ml) und RNase T1 (1000 U/ml) sowie anschließend mit Proteinase K (50 µg/ml) für 20 - 60 Minuten bei 60°C inkubiert. Aus diesem Ansatz werden die Komplexe nach bekannten Methoden der DNS-Isolierung, z. B. durch Extraktion mit Phenol und Chloroform/-Isoamylalkohol, isoliert und eine Fraktion, welche in einem Cäsiumchlorid-gradienten eine Dichte von ca. 1,82 - 1,89 g/cm³ aufweist, über einen CsCl-Dichtegradienten (Sambrook et al., Molecular Cloning, Cold Spring Harbor Laboratory, 2nd Edition 1989) angereichert. Die Fraktionen werden gegen 10 mmol/l Tris-HCl pH 7,2, 1 mmol/l EDTA dialysiert und die Komplexe mit Ethanol und Na-Acetat gefällt.

Zur Isolierung der DNS dieser Komplexe werden die Komplexe gegen 10 mmol/l Tris-HCl, 1 mmol/l EDTA, pH 7,2 dialysiert.

Die DNS wird einer "Auffüll-Reaktion" mit Hilfe der Klenow-DNS-Polymerase in Gegenwart von dNTP's nach dem Fachmann bekannten Verfahren (Sambrook et al., Molecular Cloning, Cold Spring Harbor, 2nd Edition 1989) unterzogen, anschließend in den pUC19 Vektor in Gegenwart von 0 - 10 U Ligase ligiert und das erhaltene rekombinante Plasmid in E.coli kloniert. Dabei werden bei der Replikation des Plasmids in den Bakterien proteinfreie Kopien dieses rekombinanten DNS-Moleküls hergestellt.

Auf diese Weise wurden mehrere unabhängige Plasmid-Klone zytoplasmatischen Komplexen von L929-Zellen gewonnen (pLC), und mehrere Plasmid-Klone aus der entsprechenden Fraktion von Ehrlich-Aszites-Zellen (pEFC), die zum Nachweis von neoplastisch transformierten Zellen mit unbegrenzter Proliferation geeignet sind (siehe Beispiel 2). Die entsprechenden DNS-Sequenzen sind in SEQ ID NO 1 - 46 gezeigt.

### Beispiel 2

### Vereinfachtes Verfahren zur Isolierung von Komplexen

Zellen der Maus-Tumor-Cytoplastenlinie L929 (ATCC CCL 1) werden in Gegenwart von 50 mmo/l Tris HCl pH 7,2, 10 mmol/l EDTA und 1,5 mmol/l Magnesiumchlorid durch mehrmaliges Einfrieren und Auftauen lysiert und die Zellkerne anschließend durch 30minütige Zentrifugation bei 6000 g abgetrennt. Der hierbei erhaltene Überstand wird 20 Minuten lang bei 60°C mit Proteinase K (100 µg/ml) inkubiert und anschließend auf einen Cäsiumchlorid Zweistufengradienten geschichtet, wobei die untere Fraktion (1 ml) eine Dichte von ca. 1,80 g/cm³ aufweist, die obere Fraktion (1 ml) eine Dichte von ca. 1,70 g/cm³. Nach Zentrifugation für 10 Stunden bei 150 000 g wird das Sediment in 100 µl 10 mmol/l Tris HCl pH 7,2, 1 mmol/l EDTA resuspendiert und die Menge der erhaltenen DNS in dieser Fraktion, wie in Beispiel 1 beschrieben, bestimmt.

### Beispiel 3

### Nachweis von menschlichen oder tierischen Zellen mit der Fähigkeit zu unbegrenzter Proliferation mit Hilfe der Bestimmung zytoplasmatischer Komplexe

Zum Nachweis von menschlichen oder tierischen Zellen mit der Fähigkeit zu unbegrenzter Proliferation oder zur Tumorbildung dient die Bestimmung zytoplasmatischer Komplexe in einem Lysat dieser Zellen. Zunächst werden Zytoplasten dieser Zellen nach bekannten Verfahren (Wigler und Weistein, Biochem. Biophys. Res. Comm. 63 (1975) 669 - 674) unter Verwendung von Cytochalasin B (50 µg/ml) gewonnen und durch wiederholtes Einfrieren und Auftauen lysiert. Die Mitochondrien werden aus dem Zytoplasten-Lysat mit Hilfe eines Sucrosegradienten (J. Biol. Chem. 249 (1974) 7991 - 7995) abgetrennt und die Mitochondrien-freien Fraktionen werden mit RNase A (20 µg/ml) und RNase T1 (1000 U/ml) sowie anschließend mit Proteinase K (50 µg/ml) inkubiert. Der Ansatz wird einer Gleichgewichts-Zentrifugation in einem CsCl-Dichtegradienten nach bekannten Verfahren (Sambrook et al., Molecular Cloning, Cold Spring Harbor Laboratory, 2nd Edition 1989) unterzogen. Evtl. vorhandene Komplexe werden dann über den DNS-Gehalt der Fraktionen mit einer Dichte von ca. 1,82 - 1,89 g/cm³ bestimmt (Ergebnis siehe Tabelle 1). Die Bestimmung des DNS-Gehalts erfolgt dabei photometrisch oder durch Aufnahme von Ethidiumbromid nach bekannten Verfahren (Sambrook et al.).

Es zeigt sich, daß normale Maus-Embryo-Fibroblasten (MEF), die eine begrenzte Lebenszeit haben und in Kultur nur eine bestimmte Zahl von Zellteilungen durchlaufen (etwa 15 bis 20 Passagen), keine nachweisbare DNS in diesen Fraktionen des Zytoplasmas haben. Ebenso enthalten menschliche Lymphozyten aus dem peripheren Blut (PBL), die in diesem Differenzierungsstadium nur etwa 3 - 5 Zellteilungen in vivo und in vitro durchlaufen, ehe sie dem programmierten Zelltod unterliegen, keine nachweisbaren zytoplasmatischen Komplexe. Zellen mit unbegrenzter Proliferationskapazität, wie Zellen etablierter Linien von malignen Tumoren, enthalten dagegen zytoplasmatische Komplexe in den entsprechenden Fraktionen (Tabelle 1).

**Tabelle 1**

| | | | | |
|---|---|---|---|---|
| **Nachweis von menschlichen oder tierischen Zellen mit der Fähigkeit zu unbegrenzter Proliferation mit Hilfe der Bestimmung zytoplasmatischer DNS-Protein Komplexe.** | | | | |

| Zellen | Spezies | Phänotyp der Zellen | Proliferation | zytoplasmatische DNS-Protein Komplexe [µg DNS/10⁹ Zellen] |
|---|---|---|---|---|
| PBL | Mensch | normal | transient | < 1 ng |
| WIL-2 | Mensch | Tumorzellen | unbegrenzt | ca. 200 ng |
| HS-Sultan | Mensch | Tumorzellen | unbegrenzt | ca. 100 ng |
| | | | | |
| MEF | Maus | normal | transient | < 1 ng |
| L929 | Maus | Tumorzellen | unbegrenzt | ca. 200 ng |
| EAZ | Maus | Tumorzellen | unbegrenzt | ca. 500 ng |
| Ag8.653 | Maus | Tumorzellen | unbegrenzt | ca. 300 ng |
| PBL: humane Lymphozyten aus dem peripheren Blut | | | | |
| WIL-2: humane T-Leukämie Linie (ATCC CRL 8062) | | | | |
| HS-Sultan: humane Leukämie Linie (ATCC CRL 1484) | | | | |
| MEF: Maus Embryo Fibroblasten | | | | |
| L929: neoplastisch transformierte Linie der Maus-L-Zellen (ATCC CCL 1) | | | | |
| EAZ: neoplastisch transformierte Linie des Maus Aszites Tumors (ATCC CCL 77) | | | | |
| Ag8.653: Maus Myelom-Linie (ATCC CRL 1580) | | | | |

### Beispiel 4

### Nachweis von menschlichen oder tierischen Zellen mit Fähigkeit zu unbegrenzter Proliferation oder zur Tumorbildung in einem Zell-Lysat durch Hybridisierung zytoplasmatischer Komplexe mit der pLCl08 DNS Probe (SEQ ID NO 1).

Zellen der neoplastisch transformierten Maus-Linie L929 (A) und primäre Maus Embryo Fibroblasten (MEF) (Passage 5) (B) werden in Gegenwart von 50 mmol/l Tris-HCl, pH 7.2, 10 mmol/l EDTA, 3 mmol/l MgCl₂ durch mehrmaliges Einfrieren und Auftauen lysiert. Die Zellkerne werden durch Zentrifugation bei 6000 x g für 20 Min sedimentiert. Der Überstand (Lysat des Zytoplasmas) wird mit Proteinase K (50 µg/ml) für 1 Stunde bei 60°C inkubiert. Anschließend wird die Fraktion mit KCl versetzt (ad 0,5 mol/l) und in einer Verdünnungsreihe durch einen Nitrozellulose-Filter BA85 (Schleicher & Schuell) mit Hilfe der "Minifold" Apparatur filtriert. Da die DNS-Protein-Bindung der nachzuweisenden Komplexe stabil gegenüber hohen Salzkonzentrationen und die Proteine in den Komplexen nicht durch Proteinase abbaubar sind, bleiben die Komplexe (im Gegensatz zu anderen DNS-Protein-Assoziationen) unter den gewählten Bedingungen intakt und werden im Filter zurückgehalten. Der Nitrozellulose-Filter wird schließlich zweimal mit 20 x SSC gewaschen und bei 120°C für 15 Minuten getrocknet. Zum Nachweis der Komplexe von Maus-Fibroblasten mit Fähigkeit zu unbegrenzter Proliferation wird eine Hybridisierung mit einer ³²P-markierten DNS der in SEQ ID NO 1 gezeigten Sequenz (pLC108) nach bekannten Methoden unter stringenten Hybridisierungsbedingungen (55 % Formamid, 1 mol/l NaCl, 1 % SDS, 10 % Dextransulfat, 100 µg/ml Hering Sperma DNS) bei 42°C (Sambrook et al., Molecular Cloning, Cold Spring Harbor Laboratory, 2nd Edition, 1989) durchgeführt.

### Ergebnis:

(A) Die Proben der zytoplasmatischen Lysate von L929-Zellen zeigen in Abhängigkeit von der Verdünnung des Lysats ein Hybridisierungssignal mit der Probe der SEQ ID NO 1 gezeigten Sequenz (LC108).
(B) Es wird keine Hybridisierung mit dem zytoplasmatischen Lysat primärer Maus-Fibroblasten (MEF) erhalten.

### Beispiel 5

### Nachweis von menschlichen und tierischen Zellen mit Fähigkeit zu unbegrenzter Proliferation oder zur Tumorbildung mit Hilfe der in situ Hybridisierung.

Zellen der Maus-Tumor-Linie L929 (ATCC CCL 1) (A) und primäre Maus-Embryo-Fibroblasten (Passage 5) (B) werden in DMEM-Medium auf Objektträgern kultiviert. Beide Kulturen enthalten proliferierenden Zellen, wobei die L929-Zellen unbegrenztes Proliferationspotential haben, primäre Maus-Fibroblasten dagegen stellen ihre Proliferation nach ca. 15 - 20 Passagen in Kultur ein und sterben dann ab. Weiterhin wird durch subkutane Injektion von L929-Zellen in C3H-Mäusen ein Tumor induziert, ein Gefrierschnitt von diesem Tumor angefertigt und auf einen Objektträger übertragen (C). Die Objektträger werden zweimal kurz mit PBS (Dulbecco/Vogt, J. Exp. Med. 99 (1954), 167 - 182) gewaschen, zur Fixierung mit 200 µl neutraler Paraformaldehydlösung (5 %) 5 Min. überschichtet und schließlich zweimal in 70%igem Ethanol gewaschen. Zum Nachweis von Zellen mit unbegrenztem Proliferationspotential wird das zu der in SEQ ID NO 1 gezeigten Sequenz (pLC108) homologe Oligonukleotid (Primer A): 5'GATCTTGAGTTTCCTCGTTGTAGGT3' (entsprechend Nukleotiden 1 - 25 von SEQ ID NO 1) verwendet. Dieses Oligonukleotid (100 pmol) wird in 20 µl Reaktionspuffer (20 mmol/l Kaliumkakodylat, 25 mmol/l Tris-HCl, pH 6,8, 5 mmol/l CoCl₂, 0,25 mg/ml Rinderserumalbumin) mit 25 U Terminaler Transferase (Boehringer Mannheim GmbH, Katalog Nr. 220582) 20 Min. bei 37°C mit Digoxigenin markiertem Didesoxy UTP (DIG-11-ddUTP) (1 nmol) markiert. Das markierte Oligonukleotid wird von den freien DIG-ddUTP Nukleotiden durch Gelfiltration über eine P10-Säule (Biorad) abgetrennt. Zur Hybridisierung werden die Objektträger mit den fixierten Zellen oder Gewebeschnitten mit 200 µl Hybridisierungslösung (50% deionisiertes Formamid, 4 x SSC, 10% Dextransulfat, 1 x Denhardts-Lösung, 0,25 mg/ml denaturierte tRNS, 0,5 mg/ml denaturierte Heringssperma-DNS) überschichtet. Die Lösung wird mit Deckgläschen auf dem Objektträger abgedeckt und die Objektträger 1 Stunde bei 42°C in einer feuchten Kammer inkubiert. Anschließend wird die Lösung wieder entfernt, die Zellen mit DIG-ddUTP-markierter Oligonukleotid Probe (5 ng DNS in 40 µl Hybridisierungslösung) überschichtet und über Nacht bei 42°C in einer feuchten Kammer inkubiert. Schließlich werden die Objektträger zweimal 20 Min. in 2 x SSC bei 42°C gewaschen. Zum Nachweis der hybridisierten Oligonukleotide werden die Zellen mit einem FITC-gekoppelten Anti-DIG-Antikörper (Boehringer Mannheim GmbH, Katalog Nr. 1207741) 20 Min. bei Raumtemperatur inkubiert. Nicht-gebundene Antikörper werden durch zweimaliges Waschen in PBS entfernt. Die Auswertung der Präparate erfolgt mit Hilfe der Fluoreszenzmikroskopie bei 470 nm Anregungswellenlänge.

### Ergebnis:

(A) Zellen der Tumorlinie L929 zeigen eine starke Fluoreszenz im Zytoplasma, während der Kern keine Fluoreszenz aufweist. Dabei fällt die Häufung der Hybridisierungssignale im kernnahen Bereich des Zytoplasmas besonders auf. In Kontrollexperimenten ("Scheinhybridisierung" der Zellen ohne Oligonukleotid, aber Inkubation mit dem FITC-anti-DIG Antikörper) wird kein Fluoreszenzsignal erhalten.
(B) Nach Hybridisierung primärer Maus-Embryo-Fibroblasten (MEF) wird keine Markierung der Zellen erhalten (weder im Zytoplasma noch im Zellkern).
(C) Nach Hybridisierung des Gewebeschnitts eines durch L929 Zellen induzierten Tumors zeigen Tumor-Zellen eine Fluoreszens im Zytoplasma, während das umgebende (normale) Bindegewebe und Subkutangewebe keine Hybridisierungssignale zeigt. In Kontrollexperimenten ("Scheinhybridisierung" der Zellen ohne Oligonukleotid, aber Inkubation mit dem FITC-anti-DIG Antikörper) wird kein Fluoreszenzsignal erhalten. Die zytoplasmatischen Komplexe der tumorigenen L929-Zellen mit unbegrenztem Proliferationspotential können also durch Hybridisierung mit einer erfindungsgemäßen Oligonukleotid-Probe in einem Gewebeschnitt nachgewiesen werden. Auf diese Weise können also neoplastisch transformierte Zellen in einem Gewebeschnitt oder einer Biopsie erkannt werden.

### Beispiel 6

### Nachweis von menschlichen oder tierischen Zellen mit Fähigkeit zu unbegrenzter Proliferation oder zur Tumorbildung durch Hybridisierung chromosomaler DNS.

Chromsomale DNS wird aus Zellen der Maus Linien neoplastisch transformierter Fibroblasten L929 (ATCC CCL 1), Ehrlich Aszites Tumor Zellen (ATCC CCL 77), Myelom Ag8.653 Zellen (ATCC CRL 1580), WEHI164 Fibrosarcom Zellen (ATCC CRL 1751), der permanent proliferierenden, aber nichttumorgenen Fibroblasten-Linie NIH3T3 (ATCC CRL 6473) und aus primären Maus-Embryo-Fibroblasten (MEF) (Passage 6) nach bekannten Verfahren durch Inkubation zerkleinerter Maus-Embryonen (Typ 12) mit Kollagenase (0,1 U/ml) und Dispase (0,8 U/ml) in PBS für 1 h bei 37°C gewonnen (Abken et al., J. Cell Biol. 103 (1986), 795 - 805); Sambrook et al., Molecular Cloning, Cold Spring Harbor Laboratory, 2nd Edition 1989) isoliert. Nach Spaltung der DNS mit Hind III wird die DNS elektrophoretisch aufgetrennt und im Southern Blot Verfahren auf eine Nylon-Membran übertragen. Zum Nachweis von Zellen mit unbegrenzter Proliferation wird der Blot mit einer ³²P-markierten DNS der in SEQ ID NO 29 (pEFC38) gezeigten Sequenz hybridisiert.

### Ergebnis:

Die Größen der hybridisierenden Restriktionsfragmente sind in den untersuchten Zellen unterschiedlich (Tab. 2). Dabei sind folgende Besonderheiten erkennbar:
1. Zellen mit begrenzter Lebensdauer und Proliferation (z. B. MEF) zeigen eine Hind III-Bande von ca. 3 kb und eine vcn ca. 2,6 kb.
2. Zellen mit unbegrenztem Proliferationspotential und Tumorbildung (L929, EAZ, Ag8.653, WEHI164) oder unbegrenzter Proliferation ohne Tumorbildung (NIH3T3) zeigen zusätzliche Hybridisierungssignale von Hind III-Restriktionsfragmenten mit bestimmten Größen. Dabei fehlt öfters die 3 kb-Bande. Allen unbegrenzt proliferierenden Zellen gemeinsam ist das Auftreten bestimmter zusätzlicher Hind III-Restriktionsfragmente der Größe 10 kb, 13 kb, 14,5 kb, 16,6 kb und 21,1 kb. Das Auftreten dieser zusätzlichen hybridisierenden Banden oder und/oder das Fehlen der hybridisierenden 3 kb Hind III-Bande unter Verwendung der in SEQ ID NO 29 (EFC38) gezeigten DNS-Probe zeigt somit Maus-Zellen mit unbegrenztem Proliferationspotential an.

**Tabelle 2**

| | | | | | |
|---|---|---|---|---|---|
| **Nachweis von menschlichen oder tierischen Zellen mit Fähigkeit zu unbegrenzter Proliferation oder Tumorbildung durch Hybridisierung chromosomaler DNS mit der pEFC38 DNS-Probe (SEQ ID NO 29).** | | | | | |

| Zellen | hybridisierende Restriktionsfragemente [kb] | | | | |
|---|---|---|---|---|---|
| | 1,9 kb | 2,6 kb | 3,0 kb | 6,0 kb | > 10 kb |
| MEF | - | + | + | - | - |
| NIH 3T3 | - | + | + | - | + |
| L929 | + | + | + | + | + |
| EAZ | - | + | + | + | + |
| Ag8.653 | + | + | - | - | + |
| WEHI164 | - | + | - | - | + |
| + / - Hybridisierung/keine Hybridisierung mit einem HindIII-Restriktionsfragment genomischer DNS der genannten Größe. Hind III-Restriktionsfragmente > 10 kb: 10 kb, 13 kb, 14,5 kb, 16,6 kb, 21,1 kb. | | | | | |

### Beispiel 7

### Nachweis von menschlichen oder tierischen Zellen mit Fähigkeit zu unbegrenzter Proliferation durch Amplifikation der DNS bestimmter zytoplasmatischer DNS-Protein Komplexe.

Die permanent proliferierenden Maus-Fibrom-Zellen L929 tragen zytoplasmatische DNS-Protein Komplexe, welche eine DNS enthalten, die mit der LC108 DNS (SEQ ID NO 1) hybridisieren. Maus Tumor-Zellen anderen Differenzierungsgrades, wie z. B. Ehrlich-Aszites-Tumorzellen, haben dagegen Komplexe, welche eine DNS enthalten, die mit der EFC 38 DNS (SEQ ID NO 29) hybridisiert, aber nicht mit der LC108 DNS oder einer anderen der in SEQ ID NO 1 - 20 genannten Sequenzen. Im Gegensatz zu den unbegrenzt proliferierenden Tumor-Zellen L929 und Ehrlich-Aszites-Zellen sind im Zytoplasma von primären Fibroblasten mit begrenzter Proliferationskapazität, wie z. B. Maus-Embryo-Fibroblasten, keine derartigen Komplexe nachweisbar. Mit Hilfe der Polymerase-Ketten-Reaktion (PCR) kann die DNS der jeweiligen Komplexe spezifisch amplifiziert und so die Anwesenheit permanent proliferierender Zellen in einem Zellgemisch nachgewiesen werden. Die gewählten experimentellen Bedingungen erlauben außerdem eine Unterscheidung des Differenzierungsgrades dieser Zellen, ob es sich um Fibrom-oder Aszites-Zellen handelt.

Jeweils 10⁴ Zellen primärer Maus-Embryo-Fibroblasten (Passage 6), neoplastisch transformierter Maus-Zellen der Linien L929 (ATCC CCL 1) und Ehrlich-Aszites (ATCC CCL 77) oder ein Gemisch dieser Zellen werden in 5 µl PBS resuspendiert, 15 µl H₂O hinzugegeben, sofort bei -20°C gefroren und dann 10 Min auf 95°C erhitzt. Die Proben werden dann in Gegenwart von Proteinase K (500 µg/ml) 1 Std. bei 55°C inkubiert und abschließend nochmals für 10 Min bei 95°C erhitzt. Zur Amplifikation der DNS zytoplasmatischer DNS-Protein Komplexe mit Hilfe der PCR-Technik (polymerase chain reaction) nach bekanntem Verfahren (Mullis und Fallona, Meth. Enzymol. 155 (1987): 335 - 350; Saiki et al., Science 239 (1988): 487 - 491) wird folgender PCR-Reaktionsansatz hinzugefügt:
3 µl 10x Taq-Puffer (200 mmol/l Tris-HCl, pH 8,4, 250 mmol/l KCl, 0,5% Tween 20, 1 mg/ml BSA);
0,5 µl 100 mmol/l MgCl₂ ;
1 µl dNTP-Lösung (2 mmol/l dATP, 2 mmol/l dCTP, 2 mmol/l dGTP, 2 mmol/l dTTP);
1 U Taq-Polymerase (Boehringer Mannheim);
je 100 ng Primer-Oligonukleotide A und B für die Amplifikation der LC108 homologen DNS bzw. die Primer-Oligonukleotide C und D für die Amplifikation der EFC38 homologen DNS zytoplasmatischer DNS-Protein Komplexe.

Die Primer-Oligonukleotid-Sequenzen (Primer) sind Teilsequenzen der in SEQ ID NO 1 bzw. SEQ ID NO 29 gezeigten DNS-Sequenzen.

Es werden 30 Zyklen mit folgendem Temperaturprofil durchgeführt:
1. 1 x [2 min 95°C]
2. 30 x [30 sec 55°C; 90 sec 72°C; 60 sec 95°C]
3. 1 x [15 min 72°C]

Die so amplifizierte DNS zytoplasmatischer Komplexe wird in einem 1%igen Agarose-Gel elektrophoretisch aufgetrennt und nach Inkubation mit Ethidiumbromid im UV-Licht sichtbar gemacht.

### Ergebnis:

Nach DNS-Amplifikation der LC108 homologen Komplexe mit Hilfe der Primer A und B wird eine amplifizierte DNS (203 bp) bei der Verwendung der permanent proliferierenden L929-Zellen erhalten, nicht aber bei MEF und Ehrlich-Aszites-Zellen. Nach Amplifikation der EFC38 homologen DNS wird eine amplifizierte DNS (372 bp) bei dem Lysat von Ehrlich-Aszites-Zellen, nicht aber in den Proben der MEF oder L929-Zellen erhalten (Tab. 3). Der Mischungsansatz von L929-Zellen oder Ehrlich-Aszites-Zellen in 10⁴ Maus-Embryo-Fibroblasten (MEF) zeigt, daß mit Hilfe dieser Versuchsdurchführung unbegrenzt proliferierende Zellen (L929, EAZ) in 10⁴ normalen Maus-Fibroblasten mit begrenzter Proliferationskapazität nachgewiesen werden können. Mit Hilfe der Wahl der Primer-Paare (A, B oder C,D) kann außerdem der Differenzierungsgrad der jeweiligen malignen Zellen in dem Zellgemisch bestimmt werden (Fibrom-[L929] oder Aszites-[EAZ] Zellen).

**Tabelle 3**

| | | | | | |
|---|---|---|---|---|---|
| **Nachweis von menschlichen oder tierischen Zellen mit Fähigkeit zu unbegrenzter Proliferation durch Amplifikation der DNS bestimmter zytoplasmatischer Komplexe.** | | | | | |

| Zellen/ Zellgemisch | | Anzahl der Zellen | | PCR-Amplifikation mit Hilfe der | |
|---|---|---|---|---|---|
| (a) | (b) | (a) | (b) | Primer A, B | Primer C, D |
| MEF | - | 10⁴ | - | - | - |
| L929 | - | 10⁴ | - | + | - |
| EAZ | - | 10⁴ | - | - | + |
| MEF | L929 | 10⁴ | 1 | + | - |
| MEF | EAZ | 10⁴ | 1 | - | + |
| +/- Amplifikation/keine Amplifikation der DNS zytoplasmatischer Komplexe. | | | | | |

### Beispiel 8

### Bestimmung der Anzahl menschlicher oder tierischer Zellen mit Fähigkeit zu unbegrenzter Proliferation in einem Zellgemisch durch kompetitive PCR-Amplifikation der DNS zytoplasmatischer Komplexe.

Mit Hilfe der hier aufgeführten experimentellen Anordnung kann die Anzahl der Zellen mit unbegrenzter Proliferation und bestimmten Differenzierungsgrades in einem Gemisch von normalen prä-seneszenten Zellen mit begrenzter Proliferationskapazität (annäherungsweise) bestimmt werden. Dabei wird die DNS zytoplasmatischer Komplexe mit Hilfe spezifischer Primer Oligonukleotide durch die PCR-Reaktion amplifiziert, wobei die spezifische (nachzuweisende) DNS mit einer konstanten Menge an bekannter, kompetitierender DNS konkurriert. Dadurch, daß die PCR-Reaktion in Gegenwart von DIG-dUTP erfolgt, wird die amplifizierte DNS durch modifizierte Nukleotide markiert. Die gesuchte, amplifizierte DNS der Komplexe wird dann durch Hybridisieren an eine einsträngige, homologe "Fang-Probe" gebunden, und die Menge der gebundenen, DIG-markierten DNS wird dann mit Hilfe eines anti-DIG ELISA bestimmt. Die Methode ist so ausgearbeitet, daß die experimentellen Schritte auf einer dafür vorbereiteten Mikrotiterplatte erfolgen können.

### 1. Herstellen des Zellysats

Zellgemische (insgesamt 10⁵ Zellen) aus primären Maus-Embryo-Fibroblasten (MEF) und Ehrlich-Aszites-Zellen (EAZ), welche die nachzuweisenden Ehrlich-Aszites-Zellen in steigenden Konzentrationen enthalten, werden in 100 µl 50 mmol/l Tris-HCl, pH 7,2, 10 mmol/l EDTA, 3 mmol/l MgCl₂ aufgenommen und die Zellen durch zweimaliges Einfrieren und Auftauen lysiert. Die Zellkerne werden durch Zentrifugation für 10 min bei 6000 x g sedimentiert, der Überstand mit Proteinase K (50 µq/ml) versetzt und 1 Stunde bei 55°C inkubiert. Die Proteinase wird anschließend durch Erhitzen auf 95°C für 10 min inaktiviert.

### 2. Herstellen einer spezifischen "Fangprobe"

Zum Nachweis von Ehrlich-Aszites-Zellen wird eine Fangproben-RNS verwendet, die eine Teilsequenz der in SEQ ID NO 29 gezeigten Sequenz (372 bp) aufweist. Die DNS-Sequenz (von Basenpaar 26 bis Basenpaar 350 der in SEQ ID NO 29 gezeigten Sequenz) wird in einen SP6-Vektor, z. B. pSPT18, nach bekannten Verfahren kloniert (Sambrook et al., Molecular Cloning, Cold Spring Harbor Laboratory, 2nd Edition, 1989). Mit Hilfe der SP6-RNS-Polymerase wird nach bekannten Verfahren (Dunn und Studier, J. Mol. Biol. 166: 477, 1983; Kassavetis et al., J. Biol. Chem. 257: 5779, 1982) eine einzelsträngige RNS-Probe hergestellt und gereinigt, bevorzugt mit Hilfe von Matrix-gebundener DNase I (frei von RNase). Die RNS wird mit Hilfe von Biotin-UTP und RNA-Ligase terminal markiert und von nicht-eingebautem Biotin-UTP durch Gelfiltration durch eine Sephadex G50 Säule (Biorad) gereinigt. Die Biotin-EFC38(26-346) Fangprobe steht jetzt zur Bindung an eine mit Streptavidin beschichtete Mikrotiter-Platte zur Verfügung.

### 3. Beschichten der Mikrotiter-Platte

Der Boden einer für ELISA-Tests geeigneten 96-Loch Mikrotiter-Platte wird mit Streptavidin beschichtet, gewaschen und getrocknet. Anschließend wird in 100 µl TE-Puffer (10 mmol/l Tris-HCl, pH 7,5, 1 mmol/l EDTA) die Biotin-markierte "Fangprobe" [500 ng Biotin-EFC38(26-246)] hinzugegeben und 1 Stunde bei 37°C inkubiert. Die Fangprobe bindet über Biotin an Streptavidin auf der Mikrotiterplatte. Abschließend werden die Löcher der Mikrotiterplatte mehrmals mit TE-Puffer gewaschen.

### 4. Herstellen einer EFC38-PCR-Kompetitions-Probe.

Zur kompetitiven (quantitativen) Amplifikation mit Hilfe der PCR-Technik wird eine DNS benötigt, die mit der zu bestimmenden DNS um die Bindung der spezifischen Oligonukleotid-Primer konkurriert. Dazu wird eine doppelsträngige "Kompetitions-DNS" gewonnen, die wie die in SEQ ID NO 29 gezeigte DNS 372 bp lang ist und deren Basenpaare 1 - 25 und Basenpaare 351 - 372 mit denen der in SEQ ID NO 29 gezeigten DNS identisch sind. Die Basenpaare 26 - 350 sind aber nicht identisch mit der in SEQ ID NO 29 gezeigten DNS-Sequenz, sondern weisen eine zufällige DNS-Sequenz auf, wie sie mit Hilfe handelsüblicher Oligonukleotid-Synthese-Apparaturen hergestellt werden können.

### 5. Kompetitive PCR der EFC38 homologen DNS der DNS-Protein Komplexe.

Zu dem Zellysat (100 µl) (aus 1.) wird zur Amplifikation der gesuchten EFC38 homologen DNS folgender PCR-Reaktionsansatz hinzugefügt:
12 µl 10 x Taq-Puffer (200 mmol/l Tris-HCl, pH 8,4, 250 mmol/l KCl, 0,5% Tween 20, 1 mg/ml BSA);
1,5 µl 100 mmol/l MgCl₂ ;
2,5 µl DIG-dNTP-Lösung (2 mmol/l dATP, 2 mmol/l dCTP, 2 mmol/l dGTP, 2 mmol/l dTTP, 0,7 mmol/l DIG-dUTP) (Boehringer Mannheim);
2 U Taq-Polymerase (Boehringer Mannheim);
je 100 ng Primer-Oligonukleotide C und D (siehe Beispiel 7)

Es werden 30 Zyklen mit folgendem Temperaturprofil durchgeführt:
1. 1 x [5 min 95°C]
2. 30 x [30 sec 55°C; 90 sec 72°C; 60 sec 95°C]
3. 1 x [15 min 72°C]

Abschließend werden die Proben 10 min auf 95°C erhitzt, auf 75°C abgekühlt, in die vorgewärmte Mikrotiterplatte gegeben und langsam (2°C pro min) auf Raumtemperatur abgekühlt.

### 6. Quantitative Bestimmung der amplifizierten DNS.

Die Probenlöcher der Mikrotiterplatte werden mehrfach mit je 500 µl PBS gewaschen und 1 Stunde mit einem anti-DIG--Antikörper, Fab-Fragmente, gekoppelt mit Peroxidase (150 mU/ml in PBS, 0,5 mg/ml Rinderserumalbumin) (Boehringer Mannheim) bei 37°C inkubiert. Die Probenlöcher werden zweimal mit PBS gewaschen und abschließend mit 200 µl ABTS-Substrat (Peroxidase-Substrat-Lösung) für 1 Stunde bei Raumtemperatur inkubiert. Die Extinktion des Farbniederschlags in den jeweiligen Probenlöchern wird bei 405 nm Wellenlänge mit Hilfe eines Mikroplate-Readers gemessen. Die Extinktion ist proportional der Menge PCR-amplifizierter DNS aus Komplexen und somit direkt proportional der Menge der Ehrlich-Aszites-Zellen in dem Zellgemisch.

### Beispiel 9

### Quantitativer Nachweis von Komplexen, die von Zellen mit Fähigkeit zu unbegrenzter Proliferation sezerniert werden.

Zellen mit unbegrenzter Proliferationskapazität sezernieren zytoplasmatische Komplexe. Der Nachweis dieser sezernierten Komplexe kann somit für den Nachweis von Zellen mit der Fähigkeit zu unbegrenzter Proliferation, insbesondere zum Nachweis von Tumorzellen, eingesetzt werden. In diesem Beispiel wird gezeigt, daß die sezernierten Komplexe von Ehrlich-Aszites-Zellen im zellfreien Kulturüberstand gemessen werden können. Dieses Verfahren erlaubt dabei die spezifische Bestimmung bestimmter Komplexe zur Unterscheidung von Tumor-Zellen verschiedenen Differenzierungsgrades.

### 1. Präparation des zellfreien Kulturüberstandes.

Ehrlich-Aszites-Zellen und primäre Maus Embryo Fibroblasten werden in Transwell™ (Costar, Cambridge, MA) Schalen kultiviert, die es erlauben, die Zellen in einem unteren Kompartiment zu züchten, wobei der Kulturüberstand durch eine Membran mit Poren (Durchmesser 0,3 µm) von den Zellen getrennt ist und aus einem oberen Kompartiment gewonnen werden kann. Sezernierte Komplexe diffundieren somit in das zellfreie obere Kompartiment. Es werden jeweils 10⁴, 10⁵ und 10⁶ Zellen in 3 ml Medium ausgesät und nach 2 Tagen der zellfreie Kulturüberstand (100 µl) aus dem oberen Kompartiment gewonnen. Der Überstand wird mit Proteinase K (50 µg/ml) versetzt und 1 Stunde bei 55°C inkubiert. Anschließend wird zur Inaktivierung der Proteinase die Probe 10 min auf 95°C erhitzt.

### 2. Präparation der Mikrotiterplatten, der "Fangprobe" und der Kompetitions-DNS.

Die Besichtung der ELISA-Mikrotiterplatte mit Streptavidin und die Präparation der biotinylierten Fang-Probe erfolgt wie in Beispiel 8 beschrieben, mit den dort angegebenen DNS-Sequenzen. Die Kompetitions-DNS wird ebenfalls wie in Beispiel 8 präpariert.

### 3. Kompetitive PCR-Amplifikation der DNS der zu bestimmenden Komplexe.

Der Kulturüberstand wird zu den beschichteten Mikrotiter-Löchern gegeben und mit dem in Beispiel 8 beschriebenen PCR-Reaktionsansatz versetzt. Die Amplifikation der DNS in Gegenwart von DIG-dUTP erfolgt ebenfalls wie dort beschrieben. Abschließend wird der Ansatz 10 min bei 95°C erhitzt und langsam (2°C pro min) auf Raumtemperatur abgekühlt.

### 4. Bestimmung der amplifizierten DNS.

Die Probenlöcher der Mikrotiterplatte werden mehrfach mit je 500 µl PBS gewaschen und 1 Stunde mit einem anti-DIG--Antikörper, Fab-Fragmente, gekoppelt mit alkalischer Phosphatase (20 µg/ml in PBS, 0,5 mg/ml Rinderserumalbumin) (Boehringer Mannheim) bei 37°C inkubiert. Die Probenlöcher werden zweimal mit je 500 µl PBS gewaschen. NADPH wird zugesetzt und durch die alkalische Phosphatase des gebundenen anti-DIG Antikörpers zu NADH umgesetzt. Mit Hilfe eines Enzymzyklus (AMPAK^{R}, Dako), der Alkoholdehydrogenase und Diaphorase umfaßt, wird nun NADH von dem Enzym Diaphorase nach Zugabe von INT in NAD⁺ und Formazan umgesetzt. NAD⁺ wird durch die Alkoholdehydrogenase nach Zugabe von Ethanol in Azetaldehyd und NADH umgesetzt, das dann wiederum für die Umsetzung durch die Diaphorase bereit steht. Bei jedem dieser Zyklen entsteht ein Molekül Formazan. Die Extinktion des gebildeten Farbstoffes wird bei 492 nm mit Hilfe eines Mikroplate Readers gemessen.

Die kompetitive Amplifikation der DNS der zu bestimmenden Komplexe mit Hilfe der PCR-Technik in Gegenwart von DIG-dUTP und der Nachweis der amplifizierten DNS mit Hilfe des alkalische Phosphatase-gekoppelten anti-DIG-Antikörpers sowie die anschließende Verstärkung des Signals durch einen Enzymzyklus erlaubt eine äußerst sensitiven Nachweis der zellsezernierten Komplexe. Die gemessene Extinktion ist proportional der Menge PCR-amplifizierter, DIG-markierter DNS aus sezernierten Komplexen und direkt proportional der Menge eingesetzter Ehrlich-Aszites-Zellen in der Diffusionskammer-Kultur.

### Beispiel 10

### Isolierung von DNS-Protein-Komplexen zum Nachweis von Zellen des Hodgkin-Lymphoms

Zellen der menschlichen Hodgkin-Linien HD 540 (L540) und HD 428 (L428) (Diehl et al. Cancer Treat. Rev. 66, 615-632 (1982) hinterlegt bei der Deutschen Sammlung von Mikroorganismen und Zellkulturen (DSM) Mascheroder Weg 1b, D-38124 Braunschweig am 29.06.1994 unter Nr. DSM ACC2177, (HD 540) bzw. erhältlich aus der öffentlichen Sammlung der DSM unter der Nr. DSM ACC197 (L 428)) werden in Gegenwart von 50 mmol/l Tris, pH 7,2, 10 mmol/l EDTA und 1,5 mmol/l MgCl₂ durch mehrmaliges Einfrieren und Auftauen lysiert und die Zellkerne anschließend durch 30minütige Zentrifugation bei 6.000 g abgetrennt. Der hierbei erhaltene Überstand wird 20 Minuten lang bei 60 °C mit Proteinase K (100 µg/ml) inkubiert und anschließend auf einem Cäsiumchlorid-Zweistufengradienten geschichtet, wobei die untere Fraktion (1 ml) eine Dichte von ca. 1,80 g/cm³ aufweist, die obere Fraktion (1 ml) eine Dichte von ca. 1,70 g/cm³. Nach Zentrifugation für 10 Stunden bei 150.000 g wird das Sediment in 100 µl 100 mmol/l Tris-HCl, pH 7,2, 1 mmol/l EDTA resuspendiert, gegen 10 mmol/l Tris-HCl, 1 mmol/l EDTA, pH 7,2 dialysiert und die Menge der erhaltenen DNS in dieser Fraktion, wie in Beispiel 1 beschrieben, bestimmt.

Die DNS wird einer "Auffüll-Reaktion" mit Hilfe der Klenow-DNS-Polymerase in Gegenwart von dNTPs nach dem Fachmann bekannten Verfahren (Sambrook et al., Molecular Cloning, Cold Spring Harbor, 2nd Edition, 1989) unterzogen, anschließend in den pUC19-Vektor in Gegenwart von 10 U Ligase ligiert und das erhaltene rekombinante Plasmid in E.coli kloniert. Bei der Replikation des Plasmids werden in den Bakterien proteinfreie Kopien dieses rekombinanten DNS-Moleküls hergestellt.

Auf diese Weise wurden mehrere unabhängige Plasmidklone zytoplasmatischer Komplexe von menschlischen Hodgkin-Zellen gewonnen (pHD540, pHD420). Die Insert-DNS dieser Plasmid-klone ist zum Nachweis neoplastisch transformierter Zellen des Hodgkin-Lymphoms bei sinngemäßer Anwendung der Beispiele 3 bis 9 geeignet. Die dabei zur Anwendung kommenden DNS-Sequenzen sind in SEQ ID NO 45-58 gezeigt.

Diese Sequenzen hybridisieren nicht mit den aus Mauszellen gefundenen Sequenzen.

### Beispiel 11

### Verwendung eines polyklonalen Kaninchen-Serums gegen zytoplasmatische DNS-Protein-Komplexe zum Nachweis von Maus-Tumor-Zellen

Maus-Tumor-Zellen sollen mit Hilfe eines spezifischen Antiserums gegen zytoplasmatische DNS-Protein-Komplexe nachgewiesen werden. Dazu wird ein Protein-Lysat der Zellen hergestellt, elektrophoretisch aufgetrennt, auf eine Trägermembran übertragen und mit dem spezifischen Antiserum aus Kaninchen im Western-Blot-Verfahren inkubiert. Die gebundenen Kaninchen-Antikörper werden mit Hilfe eines sekundären anti-Kaninchen-Antikörpers und der Chemolumineszenz-Reaktion nachgewiesen.

### Durchführung:

### 1. Gewinnung des Antiserums

Die Kaninchen wurden mit DNS-Protein-Komplexen (isoliert aus dem Zytoplasma von Maus L929 Zellen) durch drei s.c. Injektionen in 3 Wochen Abständen immunisiert und anschließend das Serum gewonnen.

### 2. Gewinnung eines Protein-Lysats

Die Zellen L929 (Maus-Tumor-Zellen) und MEF (Maus normale embryonale Fibroblasten) werden in Gegenwart von 50 mM Tris-HCl, pH 7.2, 10 mM EDTA, 3 mM MgCl₂, 0,2 % NP40 lysiert. Zelltrümmer, Zellkerne und Membranen werden durch Zentrifugation bei 8.000 g, 2 min. abgetrennt. Der Überstand (Protein-Fraktion) wird bei -20°C gelagert.

### 3. Western-Blot und Nachweis-Reaktion

Die Protein-Fraktion der Zellen L929 und MEF (entsprechend ca. 10⁵ Zellen) sowie isolierte DNS-Protein-Komplexe (2 µg) (als positive Kontrolle) werden in Gegenwart von SDS denaturiert, in einem SDS-Polyacrylamid Gel elektrophoretisch aufgetrennt und nach dem Fachmann bekannten Verfahren elektrophoretisch auf eine Nitrocellulose-Membran übertragen (E. Harlow, Antibodies, Cold Spring Harbor Laboratories, CSH Press). Die Membran wird TBS (20 mM Tris, 137 mM NaCl, pH 7.6), 3 % (v/v) Tween 20, 5 % (w/v) Trockenmilchpulver 1 h inkubiert, anschließend das Kaninchen-Antiserum (1:5.000) hinzugegeben und nochmals 1 h inkubiert. Die Membran wird in TBS, 3 % Tween 20 gewaschen, dann 1 h mit einem Ziege-anti-Kaninchen-Antikörper (HRPO-markiert) (1:5.000) inkubiert.

Nach erneutem Waschen wird die Detektionslösung (ECL, Amersham, RPN 2106, 2108, 2109) hinzugegeben und eine Autoradiographie angefertigt.

### Ergebnis:

Aus dem Protein-Lysat der L929 Tumorzellen reagiert das Antiserum mit Proteinen des Molekulargewichts von ca. 36 kD, 52 kD, 62 kD, 64 kD sowie mit Protein-Aggregaten von > 150 kD. Die Proteine vom Molekulargewicht 52 kD, 62 kD, 64 kD werden auch bei den isolierten DNS-Protein-Komplexen aus Tumorzellen (positive Kontrolle) durch Reaktion mit dem Antiserum gefunden. Die Bande bei 36 kD stellt wahrscheinlich Protein-Abbauprodukte dar.

In dem Protein-Lysat der MEF Normalzellen sind keine spezifischen Reaktionen mit dem Antiserum zu erkennen. Eine leichte Reaktion mit Protein-Aggregaten > 150 kD stellt wahrscheinlich eine unspezifische Bindung des Antiserums dar.

### Beispiel 12

### Isolierung von DNS-Protein-Komplexen aus Mamma-Karzinomen

Analog zur Vorgehensweise von Beispiel 10 wurden DNS-Protein-Komplexe aus Zellinie MCF 7 des Mamma-Karzinoms gewonnen. Ein Teil der Nukleinsäure ist sequenziert und in SEQ ID NOS 59-61 enthalten. Diese Sequenzen können als Sonden zur Identifizierung und Isolierung von Mamma-Karzinomzellen benutzt werden, die die erfindungsgemäßen Komplexe beinhalten. Nach in-vitro oder in-vivo Vermehrung der Nukleinsäuren kann die Gesamtsequenz der DNS dieser Komplexe bestimmt werden. Die bisher gefundenen Sequenzen hybridisieren nicht mit den aus Hodgkin- oder Maus-Zellen gefundenen Sequenzen. Ebenfalls Gegenstand der Erfindung sind also Nukleinsäuren, die ein Teil der Gesamtsequenz der Komplexe sind, bzw. deren funktionelle Äquivalente wie oben beschrieben.

## Patentansprüche

1. DNS-Protein-Komplex, welcher zum Nachweis von menschlichen oder tierischen Zellen mit Fähigkeit zu unbegrenzter Proliferation oder zur Tumorbildung geeignet ist, erhältlich durch Isolierung einer Mitochondrien-freien Fraktion des Zytoplasmas aus transformierten menschlichen oder tierischen Zellen, die in einem Cäsiumchlorid-Gradienten eine Dichte von etwa 1,82 - 1,89 g/cm³ aufweist, und Isolierung des Komplexes aus dieser Fraktion durch Extraktion mit Phenol und Fällung mit Ethanol.

2. Protein, welches zum Nachweis von menschlichen oder tierischen Zellen mit Fähigkeit zu unbegrenzter Proliferation oder zur Tumorbildung geeignet ist, erhältlich durch Behandlung eines Komplexes gemäß Anspruch 1 mit DNase I und chromatographische Isolierung des dabei freigesetzten Proteins.

3. Protein gemäß Anspruch 2, mit einem Molekulargewicht von 52 kD.

4. Protein gemäß Anspruch 2, mit einem Molekulargewicht von 62 kD.

5. Protein gemäß Anspruch 2, mit einem Molekulargewicht von 64 kD.

6. Antikörper, welcher zum Nachweis von menschlichen oder tierischen Zellen mit Fähigkeit zu unbegrenzter Proliferation oder zur Tumorbildung geeignet ist, erhältlich durch Immunisierung von Tieren mit einem DNS-Protein-Komplex gemäß Anspruch 1, einem Protein gemäß einem der Ansprüche 2 - 5 oder DNS gemäß den Ansprüchen 7 oder 8.

7. DNS, erhältlich durch Klonierung oder enzymatische Vermehrung der DNS eines Komplexes gemäß Anspruch 1 zum Nachweis von menschlichen oder tierischen Zellen mit Fähigkeit zu unbegrenzter Proliferation oder zur Tumorbildung, wobei diese DNS nicht SEQ.ID.No. 1 und nicht SEQ.ID.NO. 29 und nicht mit SEQ.ID.NO. 1 oder mit SEQ.ID.NO. 29 hybridisierende Sequenzen ist.

8. DNS gemäß Anspruch 7, welche eine der in SEQ ID NO 2 - 28 oder SEQ ID NO 30 - 45 gezeigten DNS-Sequenzen enthält oder mit einer dieser Sequenzen hybridisiert.

9. Verfahren zur Gewinnung einer DNS, welche eine der in SEQ.ID.NO. 46 - 61 gezeigten DNS-Sequenzen enthält oder mit einer dieser Sequenzen hybridisiert durch Hybridisierung einer genomischen Genbank oder eine cDNS-Bank von menschlichen Zellen mit einer der in SEQ.ID.NO. 46 - 61 gezeigten Sequenzen und Isolierung der hybridiserenden DNS.

10. Verfahren zur Gewinnung einer DNS welche eine der in SEQ.ID.NO. 2 - 28 oder 30 - 45 gezeigten DNS-Sequenzen enthält oder mit einer dieser Sequenzen hybridisiert durch Hybridisierung einer genomischen Genbank oder einer cDNS-Bank von menschlichen oder tierischen Zellen mit einer der in SEQ ID NO 2 - 28 und 30 - 45 gezeigten DNS-Sequenzen und Isolierung der hybridisierenden DNS.

11. Verfahren zur Gewinnung einer DNS, welche eine der in SEQ.ID.NO. 2 - 28 oder 30 - 61 gezeigten DNS-Sequenzen enthält oder mit einer dieser Sequenzen hybridisiert durch enzymatische Vermehrung einer DNS- oder RNS-Fraktion von menschlichen oder tierischen Zellen, wobei Oligonukleotid-Starter Moleküle verwendet werden, die mit einer der in SEQ.ID.NO. 1 - 61 gezeigten Sequenzen hybridisieren oder zu diesen oder Teilen davon homolog sind.

12. Verfahren zur Gewinnung einer DNS durch chemische Synthese einer der in SEQ ID NO 2 - 28 und 30- 61 gezeigten Sequenzen oder Teilen dieser DNS, welche zum Nachweis von menschlichen oder tierischen Zellen mit der Fähigkeit zu unbegrenzter Proliferation oder zur Tumorbildung geeignet ist.

13. Verfahren zur Gewinnung eines Proteins gemäß einem der Ansprüche 2 bis 5, dadurch gekennzeichnet, daß man einen Komplex gemäß Anspruch 1 mit DNase I behandelt und die dabei freigesetzten Proteine chromatographisch oder elektrophoretisch nach ihrer Größe auftrennt.

14. Verfahren zum Nachweis von menschlichen oder tierischen Zellen mit Fähigkeit zur Tumorbildung, dadurch gekennzeichnet, daß man Komplexe gemäß Anspruch 1 in einer Mitochondrien-freien Fraktion des Zytoplasmas einer Dichte im Cäsiumchlorid-Gradienten von ca. 1,82 - 1,89 g/cm³ bestimmt.

15. Verfahren zum Nachweis von menschlichen oder tierischen Zellen mit Fähigkeit zur Tumorbildung durch Hybridisierung einer DNS-Fraktion der zu untersuchenden Zellen mit einer DNS mit einer Sequenz gemäß SEQ ID NO 2 - 28 oder 30 - 61 oder Teilen davon, oder DNS, die mit diesen Sequenzen hybridisiert.

16. Verfahren gemäß Anspruch 15, dadurch gekennzeichnet, daß man die Hybridisierung als in-situ-Hybridisierung durchführt.

17. Verfahren gemäß Anspruch 15, dadurch gekennzeichnet, daß aus einem Lysat der Zellen oder eine DNS- oder RNS-Fraktion dieser Zellen einer Nukleinsäure enzymatisch vermehrt wird unter Verwendung von Oligonukleotiden, die mit einer DNS mit einer Sequenz gemäß SEQ ID NO 2 - 28 oder 30 - 61 oder Teilen davon hybridisieren.

18. Verfahren zum Nachweis maligner Tumoren, wobei Komplexe gemäß Anspruch 1 in Zellen einer Biopsie, eines Gewebeschnittes oder in Körperflüssigkeiten von Menschen oder Tieren nachgewiesen werden.

19. Verfahren gemäß Anspruch 18, dadurch gekennzeichnet, daß man einen Antikörper gemäß Anspruch 6 in einer Antigen-Erkennungs-Reaktion einsetzt.

20. DNS mit einer Sequenz der SEQ ID NO 2 bis 28 oder 30 bis 54 oder 56 - 61 oder Teilen davon, die als Oligonukleotid-Starter Moleküle für die enzymatische Verwahrung von DNS eines Komplexes gemäß Anspruch 1 eingesetzt werden können, oder Sequenzen, die hiermit hybridisieren und zum Nachweis von menschlichen oder tierischen Zellen mit der Fähigkeit zur Tumorbildung geeignet sind.

## Claims

1. DNA-protein complex suitable for detecting human or animal cells capable of unlimited proliferation or tumour formation which can be obtained by isolating a mitochondria-free fraction of the cytoplasm from transformed human or animal cells which have a density of about 1.82 - 1.89 g/cm³ in a caesium chloride gradient and isolating the complex from this fraction by extraction with phenol and precipitation with ethanol.

2. Protein suitable for detecting human or animal cells capable of unlimited proliferation or tumour formation which can be obtained by treating a complex of claim 1 with DNase I and chromatographically isolating the protein released in this process.

3. Protein as claimed in claim 2 having a molecular weight of 52 kD.

4. Protein as claimed in claim 2 having a molecular weight of 62 kD.

5. Protein as claimed in claim 2 having a molecular weight of 64 kD.

6. Antibodies suitable for detecting human or animal cells capable of unlimited proliferation or tumour formation which can be obtained by immunizing animals with a DNA-protein complex as claimed in claim 1, a protein as claimed in one of the claims 2 - 5 or DNA as claimed in claims 7 or 8.

7. DNA suitable for detecting human or animal cells capable of unlimited proliferation or tumour formation which can be obtained by cloning or enzymatically amplifying the DNA of a complex of claim 1, wherein this DNA is not SEQ ID NO. 1 and not SEQ ID NO. 29 and is not a sequence which hybridizes with SEQ ID NO. 1 or with SEQ ID. NO. 29.

8. DNA as claimed in claim 7 which contains one of the DNA sequences shown in SEQ ID NO. 2 - 28 or SEQ ID NO. 30 - 45 or hybridizes with one of these sequences.

9. Method for obtaining a DNA which contains one of the DNA sequences shown in SEQ ID NO. 46 - 61 or hybridizes with one of these sequences by hybridizing a genomic gene bank or a cDNA bank of human cells with one of the sequences shown in SEQ ID NO. 46 - 61 and isolating the hybridizing DNA.

10. Method for obtaining a DNA which contains one of the DNA sequences shown in SEQ ID NO. 2 - 28 or SEQ ID NO. 30 - 45 or hybridizes with one of these sequences by hybridizing a genomic gene bank or a cDNA bank of human or animal cells with one of the sequences shown in SEQ ID NO. 2 - 28 and 30 - 45 and isolating the hybridizing DNA.

11. Method for obtaining a DNA which contains one of the DNA sequences shown in SEQ ID NO. 2 - 28 or SEQ ID NO. 30 - 61 or hybridizes with one of these sequences by enzymatically amplifying a DNA or RNA fraction of human or animal cells using oligonucleotide starter molecules which hybridize with one of the sequences shown in SEQ ID NO. 1 - 61 or are homologous to these sequences or parts thereof.

12. Method for obtaining a DNA by chemically synthesizing one of the sequences shown in SEQ ID NO. 2 - 28 and 30 - 61 or parts of this DNA which is suitable for detecting human or animal cells capable of unlimited proliferation or tumour formation.

13. Method for obtaining a protein as claimed in one of the claims 2 to 5, wherein a complex as claimed in claim 1 is treated with DNase I and the released proteins are chromatographically or electrophoretically separated according to size.

14. Method for detecting human or animal cells capable of tumour formation, wherein complexes as claimed in claim 1 are determined in a mitochondria-free fraction of the cytoplasm which has a density 1.82 - 1.89 g/cm³ in a caesium chloride gradient.

15. Method for detecting human or animal cells capable of tumour formation by hybridizing a DNA fraction of the cells to be examined with a DNA having a sequence of SEQ ID NO. 2 - 28 or 30 - 61 or parts thereof, or DNA which hybridizes with these sequences.

16. Method as claimed in claim 15, wherein the hybridization is carried out as an in-situ hybridization.

17. Method as claimed in claim 15, wherein a nucleic acid is enzymatically amplified from a lysate of the cells or from a DNA or RNA fraction of these cells using oligonucleotides which hybridize with a DNA having a sequence of SEQ ID NO. 2 - 28 or 30 - 61 or parts thereof.

18. Method for detecting malignant tumours, wherein complexes as claimed in claim 1 are detected in cells of a biopsy, a tissue section or in body fluids of humans or animals.

19. Method as claimed in claim 18, wherein an antibody as claimed in claim 6 is used in an antigen recognition reaction.

20. DNA having a sequence of SEQ ID NO. 2 to 28 or 30 to 54 or 56 - 61 or parts thereof which can be used as oligonucleotide starter molecules for the enzymatic amplification of DNA of a complex as claimed in claim 1, or sequences which hybridize with it and are suitable for detecting human or animal cells capable of tumour formation.

## Revendications

1. Complexe d'ADN-protéine qui est approprié pour l'identification de cellules humaines ou animales susceptibles de proliférer de manière illimitée ou de générer des tumeurs, que l'on obtient via isolation d'une fraction du cytoplasme exempte de mitochondries, provenant de cellules humaines ou animales transformées, qui présente, dans un gradient de chlorure de césium, une densité d'environ 1,82-1,89 g/cm³, et via isolation du complexe de cette fraction par extraction dans du phénol et par précipitation dans de l'éthanol.

2. Protéine qui est appropriée pour l'identification de cellules humaines ou animales susceptibles de proliférer de manière illimitée ou de générer des tumeurs, que l'on obtient par traitement d'un complexe selon la revendication 1 avec de la désoxyribonucléase I et par isolation chromatographique de la protéine libérée en l'occurrence.

3. Protéine selon la revendication 2, possédant un poids moléculaire de 52 kD.

4. Protéine selon la revendication 2, possédant un poids moléculaire de 62 kD.

5. Protéine selon la revendication 2, possédant un poids moléculaire de 64 kD.

6. Anticorps qui est approprié pour l'identification de cellules humaines ou animales susceptibles de proliférer de manière illimitée ou de générer des tumeurs, que l'on obtient par immunisation d'animaux avec un complexe d'ADN-protéine selon la revendication 1, avec une protéine selon l'une quelconque des revendications 2 à 5 ou avec de l'ADN selon les revendications 7 ou 8.

7. ADN que l'on obtient par clonage ou par multiplication enzymatique de l'ADN d'un complexe selon la revendication 1 pour la détection de cellules humaines ou animales susceptibles de proliférer de manière illimitée ou de générer des tumeurs, dans lequel cet ADN représente des séquences qui ne s'hybrident pas à SEQ.ID.NO. 1 et à SEQ.ID.NO. 29 et qui ne s'hybrident pas à SEQ.ID.NO. 1 ou à SEQ.ID.NO. 29.

8. ADN selon la revendication 7, qui contient une des séquences d'ADN représentées dans SEQ.ID.NO. 2 - 28 ou SEQ.ID.NO. 30 - 45 ou qui s'hybride à une de ces séquences.

9. Procédé pour l'obtention d'un ADN qui contient une des séquences d'ADN représentées dans SEQ.ID.NO. 46 - 61 ou qui s'hybride à une de ces séquences, via hybridation d'une banque génomique ou d'une banque d'ADNc de cellules humaines à une des séquences représentées dans SEQ.ID.NO. 46 - 61 et via isolation de l'ADN qui s'hybride.

10. Procédé pour l'obtention d'un ADN qui contient une des séquences d'ADN représentées dans SEQ.ID.NO. 2 - 28 ou 30 - 45 ou qui s'hybride à une de ces séquences, via hybridation d'une banque génomique ou d'une banque d'ADNc de cellules humaines ou animales à une des séquences d'ADN représentées dans SEQ.ID.NO. 2 - 28 et 30 - 45 et via isolation de l'ADN qui s'hybride.

11. Procédé pour l'obtention d'un ADN qui contient une des séquences d'ADN représentées dans SEQ.ID.NO. 2 - 28 ou 30 - 61 ou qui s'hybride à une de ces séquences, via multiplication enzymatique d'une fraction d'ADN ou d'ARN de cellules humaines ou animales, dans lequel on utilise des molécules d'amorçage oligonucléotidiques qui s'hybrident à une des séquences représentées dans SEQ.ID.NO. 1 - 61 ou qui sont homologues à ces dernières ou à des parties de ces dernières.

12. Procédé pour l'obtention d'un ADN par synthèse chimique d'une des séquences représentées dans SEQ.ID.NO. 2 - 28 et 30 - 61 ou de parties de cet ADN qui est approprié pour l'identification de cellules humaines ou animales susceptibles de proliférer de manière illimitée ou de générer des tumeurs.

13. Procédé pour l'obtention d'une protéine selon l'une quelconque des revendications 2 à 5, caractérisé en ce qu'on traite un complexe selon la revendication 1 avec de la désoxyribonucléase I et on sépare, en fonction de leur taille, les protéines libérées en l'occurrence, par voie chromatographique ou par voie électrophorétique.

14. Procédé pour l'identification de cellules humaines ou animales susceptibles de générer des tumeurs, caractérisé en ce qu'on détermine des complexes selon la revendication 1 dans une fraction du cytoplasme exempte de mitochondries, dont la densité dans le gradient de chlorure de césium s'élève d'environ 1,82 à 1,89 g/cm³.

15. Procédé pour l'identification de cellules humaines ou animales susceptibles de générer des tumeurs, par hybridation d'une fraction d'ADN des cellules à identifier à un ADN possédant une séquence selon SEQ.ID.NO. 2 - 28 ou 30 - 61 ou des parties de ces dernières, ou à un ADN qui s'hybride à ces séquences.

16. Procédé selon la revendication 15, caractérisé en ce qu'on effectue l'hybridation sous la forme d'une hybridation in situ.

17. Procédé selon la revendication 15, caractérisé en ce qu'on procède à une multiplication enzymatique d'un acide nucléique à partir d'un lysat des cellules ou d'une fraction d'ADN ou d'ARN de ces cellules, en utilisant des oligonucléotides qui s'hybrident à un ADN possédant une séquence selon SEQ.ID.NO. 2 - 28 ou 30 - 61 ou des parties de ces dernières.

18. Procédé pour l'identification de tumeurs malignes, dans lequel on identifie des complexes selon la revendication 1 dans des cellules d'une biopsie, d'une coupe histologique ou dans des liquides corporels d'êtres humains ou d'animaux.

19. Procédé selon la revendication 18, caractérisé en ce qu'on met en oeuvre un anticorps selon la revendication 6 dans une réaction de reconnaissance d'antigènes.

20. ADN possédant une séquence des SEQ.ID.NO. 2 à 28 ou 30 à 54 ou 56 à 61 ou des parties de ces dernières, que l'on peut mettre en oeuvre à titre de molécules d'amorçage oligonucléotidiques pour la multiplication enzymatique de l'ADN d'un complexe selon la revendication 1, ou séquences qui s'hybrident à ce dernier et qui sont appropriées pour l'identification de cellules humaines ou animales susceptibles de générer des tumeurs.


### SEQUENZPROTOKOLL

### (1) ALLGEMEINE ANGABEN:

(i) ANMELDER:
   (A) NAME: BOEHRINGER MANNHEIM GMBH
   (B) STRASSE: Sandhofer Str. 116
   (C) ORT: Mannheim
   (E) LAND: DE
   (F) POSILEITZAHL: 68305
   (G) TELEFON: 0621/759-4348
   (H) TELEFAX: 0621/759-4457
(ii) BEZEICHNUNG DER ERFINDUNG:
   Verfahren zur Identifizierung von menschlichen und tierischen Zellen mit der Faehigkeit zu unbegrenzter Proliferation oder zur Tumorbildung
(iii) ANZAHL DER SEQUENZEN: 62
(iv) COMPUTER-LESBARE FASSUNG:
   (A) DATENTRÄGER: Floppy disk
   (B) COMPUTER: IBM PC compatible
   (C) BETRIEBSSYTEM: PC-DOS/MS-DOS
   (D) SOFTWARE: PatentIn Release #1.0, Version #1.30B (EPA)

### (2) ANGABEN ZU SEQ ID NO: 1:

(i) SEQUENZKENNZEICHEN:
   (A) LÄNGE: 203 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 1:
   GATCTTGAGT TTCCTCGTTG TAGGTCTTCC CTGGCTCTGC TCACGCTCTC ACTGACTTCT    60
   CTCAGCTCAG TCACAGTGTC TATTTCTTTC CACTTAAAGA TGTGCATTTT TATTTGATGC   120
   GTGCAGGTGT TTTGCCTGCA TGGATGGCTG TGCACCATGT ATGGGACCTG GTGCTCTTGG   180
   AGGCCAGCAG AGGGCTTTGG ATC                                           203

### (2) ANGABEN ZU SEQ ID NO: 2:

(i) SEQUENZKENNZEICHEN:
   (A) LÄNGE: 69 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 2:
   GATCTTTTCT TGAAAGTACG TAAGCCTGGG GTTTTAATTC TCATCTTAGA ACACAGTGTA 60
   AAAAGGATC 69

### (2) ANGABEN ZU SEQ ID NO: 3:

(i) SEQUENZKENNZEICHEN:
   (A) LÄNGE: 224 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 3:
   GATCTTCAGT GTCCATACAC TCAGATTCCT GATTAAATCA AAGCATCAGA ACCACTCCCC 60
   CTGCAAAATG TCCCAAAATG TAAAATCAAT TGATGTACAA CTCAGAATAC ACCACTCTGA 120
   GGCATATTTT CCAGGGACTC TAACCTACTT CAGAAGTACA TGGTACTTGC TTCTCTATTA 180
   CAGCATAATA GAGATGAGCA ACATAGTGCA CTTACAAACA GATC 224

### (2) ANGABEN ZU SEQ ID NO: 4:

(i) SEQUENZKENNZEICHEN:
   (A) LÄNGE: 50 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 4:
   GATCAGACCT CTACCTTCAC CCATGAGGCT TGCTTGCAGC AATTAAGATC 50

### (2) ANGABEN ZU SEQ ID NO: 5:

(i) SEQUENZKENNZEICHEN:
   (A) LÄNGE: 55 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 5:
   GATCTTTAGC TCCCTGGATA CTTTCTCTAG CTCCTCCATT GGGGGCCCTG TGATC 55

### (2) ANGABEN ZU SEQ ID NO: 6:

(i) SEQUENZKENNZEICHEN:
   (A) LÄNGE: 72 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 6:
   GATCTTTCCA AGACTTTTAT CATGAATGGG TGTTGGATCT TGTCAAATGC TTTTTCTGCA 60
   TCCAACGAGA TC 72

### (2) ANGABEN ZU SEQ ID NO: 7:

(i) SEQUENZKENNZEICHEN:
   (A) LÄNGE: 47 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 7:
   GATCCTGGCA TTACCCTATC CTGCATTAAG GGGGGAACAG GAAGATC 47

### (2) ANGABEN ZU SEQ ID NO: 8:

(i) SEQUENZKENNZEICHEN:
   (A) LÄNGE: 34 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 8:
   GATCCATTCT ATCTCCACCC CCACCATGAG GATC 34

### (2) ANGABEN ZU SEQ ID NO: 9:

(i) SEQUENZKENNZEICHEN:
   (A) LÄNGE: 176 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 9:
   GATCTTCGCC GCCGACCTGA CCTCGATCGA GGTGGCGCNN GGCGTCGATC ATATCATCTT 60
   TGGCTGGGTG TTCTTCGCGC TGGTGATGGC AGCGGTGCTG GCCATCGGCT GGCAGTTTTT 120
   CGATCGTTCC CCCGATGATC CCATCACTGG CCAGGCATGG AGGCCCACAG GGGATC 176

### (2) ANGABEN ZU SEQ ID NO: 10:

(i) SEQUENZKENNZEICHEN:
   (A) LÄNGE: 43 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 10:
   GATCACGCCG GACGAAGCCT ACGACATCAC CGTCTACCAG ATC 43

### (2) ANGABEN ZU SEQ ID NO: 11:

(i) SEQUENZKENNZEICHEN:
   (A) LÄNGE: 42 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 11:
   GATCTCCTCG CCTCTCTATC CCCAGCACCT GCCAAGAGGA TC 42

### (2) ANGABEN ZU SEQ ID NO: 12:

(i) SEQUENZKENNZEICHEN:
   (A) LÄNGE: 110 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 12:
   GATCTGGTCG ATGCCGATTG CTGAGGTTGG TTGGTGCGGT GATTCCTGTT AATTTCCTTA 60
   GAGGAGGGTG GGTTTGCAGT GTCATGAGAA TGGAGGGTCG GGATTTGATC 110

### (2) ANGABEN ZU SEQ ID NO: 13:

(i) SEQUENZKENNZEICHEN:
   (A) LÄNGE: 70 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 13:
   GATCAAAAAG CGTGGAAAAA TTTGAATGTC ATGTCGAGCT GGTATCGGCT TCTGGAATAC 60
   CGAAAGGATC 70

### (2) ANGABEN ZU SEQ ID NO: 14:

(i) SEQUENZKENNZEICHEN:
   (A) LÄNGE: 88 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 14:
   GATCTGGGGC CAGTGATAGC CATCTCTGTN CCTGTATGTG CCCGGACATC CTGGGGATAA 60
   AGAGAGATCC TTCATGTGGA GGAAGATC 88

### (2) ANGABEN ZU SEQ ID NO: 15:

(i) SEQUENZKENNZEICHEN:
   (A) LÄNGE: 75 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCREIBUNG: SEQ ID NO: 15:

### (2) ANGABEN ZU SEQ ID NO: 16:

(i) SEQUENZKENNZEICHEN:
   (A) LÄNGE: 94 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 16:
   GATCCGTCGT CGCTGCCGTG CRCCGCCTTG CCATGGGAAC CGTGTCTGGG CCCTGTGTCA 60
   CAAGGTTCCC CCCGGGATGA CAACCGTTGT GATC 94

### (2) ANGABEN ZU SEQ ID NO: 17:

(i) SEQUENZKENNZEICHEN:
   (A) LÄNGE: 47 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 17:
   GATCTAACCG TGGAAGGCAG CGCCAGAGAG ACTGAATCAG GAGGATC 47

### (2) ANGABEN ZU SEQ ID NO: 18:

(i) SEQUENZENNZEICHEN:
   (A) LÄNGE: 94 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 18:
   GATCTGAGTT CGAGGCTAGT CTGGTCTACA GAATAAGTTC CAGGACAACC AGGGCTGCAC 60
   AGAGAAATCC TGCCTCCGGA AAGGAAAAAA GATC 94

### (2) ANGABEN ZU SEQ ID NO: 19:

(i) SEQUENZKENNZEICHEN:
   (A) LÄNGE: 202 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 19:
   GATCTTGAGT TTCCTCGTTG TAGGTCTTCC CTGGCTCTGC TCACGCTCTC ACTGACTTCT 60
   CTCAGCTCAG TCACAGTGTC TATTTCTTTC CACTTAAAGA TGTGCATTTT TATTTGATGC 120
   GTGCAGGTGT TTTGCCTGCA TGGATGGCTG TGCACCATGT ATGGGCCTGG TGCTGTTGGA 180
   GGCCAGCAGA GGGCTTTGGA TC 202

### (2) ANGABEN ZU SEQ ID NO: 20:

(i) SEQUENZKENNZEICHEN:
   (A) LÄNGE: 80 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCREIBUNG: SEQ ID NO: 20:

### (2) ANGABEN ZU SEQ ID NO: 21:

(i) SEQUENZKENNZEICHEN:
   (A) LÄNGE: 59 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 21:
   GATCTGCTCT ATCTCAAATG GCATTCATTC CAGGCAGCCT ATGAAAGGAA CACTTGATC 59

### (2) ANGABEN ZU SEQ ID NO: 22:

(i) SEQUENZKENNZEICHEN:
   (A) LÄNGE: 233 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 22:
   GATCAGCTCT CCTGCTCTCA GGACCCTAGG GCCAGGTCTC CTACCTGCAG CAGGTTATGA 60
   GGAGTAAAGG GGTCTACCCC ACCATAAGGA GATGAGTATG CCAGTTCCCA TGTTCACATT 120
   CTTGAGGACA GCTCACTTGT ATCTCCCATG TGAGGGGCTC ACTCTAAGGA GTATTACAAC 180
   TTGACTGTCA GGTGGCATCC AAGCATCTCT CTGCTGCTAC ATGGCCAAGG ATC 233

### (2) ANGABEN ZU SEQ ID NO: 23:

(i) SEQUENZKENNZEICHEN:
   (A) LÄNGE: 282 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 23:
   GATCTCAGCT TTTGATAGAC TGAGGAAGAA GGGCCTCCTA GGGTACAAAT GTAAGTTTTA      60
   TTCACCCGAG GTAATTTCCA GCTGCTAACC TGGAAGCTTC TACCCTCAGT AAAATCTTAC     120
   CTAGACCTAG AATGTTTTTC AGCCTAAGAC TTATTGCAGA ATAATGCTCA CCCTTTCTAG     180
   GTTCATTCTG TGCTGACTGG TCAACTCAGC TATGCAAATT GCTGACTGAC TTAAGCAGGT     240
   TCTTCAGCTT GGACTGACGG CCCGCTTGGC CTCAGACTAC TT                        282

### (2) ANGABEN ZU SEQ ID NO: 24:

(i) SEQUENZKENNZEICHEN:
   (A) LÄNGE: 125 Basenpaare
   (B) ART: Nucleotid
   (C) STRANGFORM: Einzelstrang
   (D) TOPOLOGIE: linear
(xi) SEQUENZBESCHREIBUNG: SEQ ID NO: 24:
   GATCTTCATA AACGCGGGGT TCGGTCCCAG GGCTGGCACT CTGTCGTAGT ACCCCACCGA      60
   GACCCCTATT AGGCCAATAC AGCACCGCGN NNCTTCCTTT TCCCCACCCC ACCCGGCCTA     120
   TAGTT                                                                 125

### (2) ANGABEN ZU SEQ ID NO: 25:

(i) SEQUENZKENNZEICHEN:
   (A) LÄNGE: 118 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 25:
   GATCTTTGGC GCTGCGCAGA AGCCCAGTCC GAGGCGCGCG TCTTCGACCG GGACCGTGCT    60
   CCGCGGCGNG CTGCCAGCCA AAGCCCAGGT CGACAGGTAG CCGTCGCGGA ACTCGATC     118

### (2) ANGABEN ZU SEQ ID NO: 26:

(i) SEQUENZKENNZEICHEN:
   (A) LÄNGE: 500 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 26:
   GATCTGAGCA AAAGTTTTCA AAATTCTGGA ATTAGAAAAT TTGAAGGTCG AAAATACTTA    60
   TATGGAACAT GAAGGACTGT GCTTTACACT TATGTGGTTT GTTGTTGTTT ATCTGTTATG   120
   GTTTTGTTTT TGCTTTGTTT TGTTTTTCTG GTGCTGCACA TAGGCCCTGA AGAAGCTTGG   180
   TTGCCGAGAC AAGCCACCAC AGCACACGGA ACTCCAGGGC TACTCTGATC CTATTTTTTC   240
   ATCCATTCAG TTAGCCTGAA TCTTTTTTAT TAGGGAATTG AGTCCATTGA TGTTGAGAGA   300
   TATTAATGAC CAATGATTGT TAATTCCTGT TATTTTGATG GTGGTGGTAG TAGTGTGTGT   360
   GTGTGTATGT GTGTGTGTAG TATTTCCCTT CTTTTGGTTT TGCTGATGTG ATATTTATTT   420
   CATGTTTTCA TGGGTGTACT TAATCTTCTT GGGTTGGAGT TTTCCTTCTA CTAGGGATAA 480
   ATTTGTGGAC AGATATGGTT 500

### (2) ANGABEN ZU SEQ ID NO: 27:

(i) SEQUENZKENNZEICHEN:
   (A) LÄNGE: 592 Basenpaare
   (B) ART: Nucleotid
   (C) STRANGFORM: Einzelstrang
   (D) TOPOLOGIE: linear
(xi) SEQUENZBESCHREIBUNG: SEQ ID NO: 27:
   GATCCCGGAC TAGGATTAGC CTGTCTATGC CAGCAAATGA GTCAGGCAAC TGATGGTGAG 60
   TCCTTCAGGT GTATATGTGT GGACAATTAA TTTTTATTAA AAAAATAAAT AAAGCCAAAG 120
   GGAAATGTAT ATTTTAATGA CCTAGGGTAT TAATTTTTTT GAAAGTCACA CATCAAAGAC 180
   TAGGAAAAGC CACCAACACC CTAAGAATGT GATTTTATCC TTTTTCCTCT GTGTGGGATG 240
   TGTTTTAGTT TAGGTTTCTT TCTGTTTCAA TTTGGACCTG TAGAGGAATG ACCGTCACTG 300
   TCTTATTCTG GAGGGAAGTC CCTGATAGTG CCCCACTACA CACACACACA CACACATACA 360
   CACACACAAA TTAGGTATTA AAATATGGTT TTAAGAGTTT TAAAAATGGA TTAGCAGTGA 420
   TTCAATTAAA ATTAGAAAAG AACAACAACA AATATGTGAG AGACTTGCCT CTCAGTGACC 480
   CCAGGCCCTG TCAGCTGATA GTGGGTGGTA ACCAGACAGC CCATTTCTGT GTCGATAGAT 540
   GAACTGTGGG AGAGTGACTC CAGAAAGAAC CAACCAGTGA GGGGTCGAGA TC 592

### (2) ANGABEN ZU SEQ ID NO: 28:

(i) SEQUENZKENNZEICHEN:
   (A) LÄNGE: 255 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 28:
   GATCCCTTTA GCTCCTTGGG TTCTTTCTCT AGCTCCTCCA TTGGGAGCCC TGTGATCAGC 60
   CTGTACCACA TAGCAAATGT CAGGCCTCTT GGGGGAGTAT TGCAAGATAT TGCTACAAAT 120
   AACAACAAAA GCAAACAAAG TTAACGTTTG CATAGCAACC TCCACCCCCC CAAGATTATA 180
   CCTGTACATA ATTTATGTTC AAAGACAGTG GCACAGGTCT GGCAAGATGC CTCACTTGAG 240
   AGAAGCTCAC AGATC 255

### (2) ANGABEN ZU SEQ ID NO: 29:

(i) SEQUENZKENNZEICHEN:
   (A) LÄNGE: 372 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 29:
   GATCCAATCA GCTCAGCCAC CCCCAGCTCT CCTGTATGTA TGGCTCCAAT GCTGTTCATC 60
   CCTCAGCATA AATCAATCAT TTGGTTTAGA TTCCTCCCTT TGACTTATTG CTACTATTAG 120
   TATCAGTGAC TCTTCAGCCG ATTCTTTTCA GACATTGGAA CCCCAGCCTC AGATCACAGT 180
   TGTAGAACAA ATATTAAAAG AGTAAATTAT TATATCATTG AACATTCAAA AGTGCTTTGC 240
   AGTCATTGAC ACATAATAAT AATGAAGCCT AAACAGTAAC ATGAAAATGT GGAATTGTAT 300
   TAATGTAAAA TCAAGGCCTG GGGNATAGCT CATTGGTGGA TGTTTGGCTA TCATGTGGGA 360
   GGGCCTGGTT TT 372

### (2) ANGABEN ZU SEQ ID NO: 30:

(i) SEQUENZKENNZEICHEN:
   (A) LÄNGE: 128 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 30:
   GATCTTGTCA AATGCTTTTT CTGCATCCAA CGAGATGATC CAACACCCAT TCATGATAAA 60
   AGTCTTGGAA AGATCATGGC GACCACACCC GTCCTGTGGA TCCACAGGAC GGGTGTGGTC 120
   GCATGATC 128

### (2) ANGABEN ZU SEQ ID NO: 31:

(i) SEQUENZKENNZEICHEN:
   (A) LÄNG: 184 Basenpaare
   (B) ART: Nucleotid
   (C) STRANGFORM: Einzelstrang
   (D) TOPOLOGY: linear
(ii) ART DES MOLEKÜLS: Genom-DNA
(xi) SEQUENZBESCHREIBUNG: SEQ ID NO: 31:

### (2) ANGABEN ZU SEQ ID NO: 32:

(i) SEQUENZKENNZEICHEN:
   (A) LÄNGE: 372 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 32:
   GATCTAATAC CAATACTCTT CAAACTATTC CACAAAATAG AAACAGAAGG AACACTACCC 60
   ATTCATTCTA TGAAGCCACA TTTTTGCCAT GTATTCTCCC TTCAAAATGG AAAGGTTTCT 120
   GTCTAATCAG GAACTTGTCA CAATTTCCTT TCTTGGAGGA CTTCATAAGA GATTTTTTTC 180
   TACTTCTACC ACATTTAAGA TTCCAAACAG ATTTTAAACG GTTGTGTTCA TATTACTTTA 240
   GTTCAGAAGA TATCATGTAT ATATAAGAGG CATTTAACAA TTATAAATTA TTTGGATGAC 300
   TTAAAAATAT CAATACTGAG TTGTATATTT TAAAATAAAT TTTATTGGTT TTAAAAAAAC 360
   AACCATAGGA TC 372

### (2) ANGABEN ZU SEQ ID NO: 33:

(i) SEQUENZKENNZEICHEN:
   (A) LÄNGE: 184 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 33:
   GATCTCAGTT TTTAAAAATA AAGAAAATGC ATTCTTTTCA GAAGGAACAT GAAGATCTAG    60
   AAGAAGTTCT GTGGCTAGAT GGATTTTGAA AGGTCCCGAA AGGTCCCGAA AGGTCCGGGG   120
   GNNGCCTGCT TCATGACTGT CACACACTTC AGTTGTCCAG GGAAGAGATA GAAAATGTGT   180
   GATC                                                                184

### (2) ANGABEN ZU SEQ ID NO: 34:

(i) SEQUENZKENNZEICHEN:
   (A) LÄNGE: 385 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 34:
   GATCTCGCCT TGCAGATTTT TTTACCAACT GCCAGCCAGA GTCAAGGTCT GTCAGCAACT 60
   GTCTTAAGGA GAACTACGCA GACTGCCTCC TGGCCTACTC GGGACTGATT GGTAAGACCA 120
   GGGGAGGGAG GGAATGGCAA AGAAATCTGG ATTACAGATC AAGATGGGAC AGTTTAGCTT 180
   CTGGTGACAG GCTAGATCCG ATGCCCTCTT CTGGTGTGTC TCAAGACAGC TACAGTGTAC 240
   TTATATACAT AAAGTAAATA AATATTTTAA ACAAAACTTT TAAAAAAAAA AAGATTGGCT 300
   ACAAAACTGA CCTATAGGGC TCCTAGCAGT CCTTGTCACC ACATAGCTGC AAACCCACCT 360
   AGGGGAAAAC TGAGGCAGGA GGATC 385

### (2) ANGABEN ZU SEQ ID NO: 35:

(i) SEQUENZKENNZEICHEN:
   (A) LÄNGE: 371 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 35:
   GATCTAACTT CCCTGTCCTC ATTGCTTTCA TGTAGAAACA TTCTGCAGCC AGAAGTGGCA 60
   GCACACAGCC CCGGTCCCCA CATCTGGGAG TCTGAGATAG ATGGAGCCTA AGTTTGAGAA 120
   CTAAGTACTT TACAATTGCA GGCATAATTG AAATTGAGCT GCCTTCTGTG TCTTCTGGAC 180
   AAAGTTAACG TGATTTATAG GAAGGCATTC TGTGATCCTT TTCTGGTTGC ACCATCCTGT 240
   TCTCTCCAGG CCTGTGAATC ACAGCACACC AGTCCACATG TGCCTGCGCT GCTCTGTTGT 300
   GGCAAGTGCT CCATCAGACT GGGATTTCTC TGCCAGGAAG GCAGGCTGGT CCCAGGCTAC 360
   TTAGGAAGAT C 371

### (2) ANGABEN ZU SEQ ID NO: 36:

(i) SEQUENZKENNZEICHEN:
   (A) LÄNGE: 396 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 36:
   GATCTAGAGA GGTTAGGTAA AGAGTAGAGC TATAAATATA AGCCTGGGTT TCCCTGGGAA    60
   GGAGTATTAA TTAGACTAGA TTCTCCATGA GCTGGGGAAG GGGGTAGAGG GNNGGGGAAG   120
   GGAAGAAGAC AGGAACAGGA GAGATATTGT GTGTATGTAT GTGGTGGGGT GGTGGAGGGA   180
   AAGAGTGTAG NNGACTGGCA GTCAGATTGG GAGGCATTGA GTGATGTAGA ACTAGTGTGG   240
   TAGAACTTGT GACTCTCTGA GTGTAACACT ATTGCAGACT CACTAATGGA GGATTCAGAG   300
   TCTGATTTGT CACTTTTTGT AGTCAGGCAA GGCTTCCAGC GGAAGCTGTG GGCTACACTT   360
   GATTGCGCTG TTGGCTGAGG AGGACCCATG GAGATC                             396

### (2) ANGABEN ZU SEQ ID NO: 37:

(i) SEQUENZKENNZEICHEN:
   (A) LÄNGE: 433 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 37:
   GATCCAAGGG AGTGTTCCAG TTTTTCTGGA AGCTGAGATG CTCCAGCTGG TAGCTTGAGC 60
   TTCACAAAGA TGCTGCCCTA ATAAATGTCA AGCACCCAGG GACTTCTCAG GAGCACATGC 120
   TATGCTTAAA GGGGCCACAG TGAAATCTTA GACAAAGATG GATGGAGGTC TTGGCTTCTT 180
   TTGTCTGTCT CTGTCTCATC TTTCTCTCTC TCTCTGTGGC CTGTATGTAT GCATGTGCCT 240
   TGTGCCAAGG AGGGCAGAAA AGGGTGTTAG ATCCTCCTGC CTCAGTCTTC TACATGCTGG 300
   TTTTGAAGGC TGGTGCCACC ATTCCCAGCT CTCAAACTTG CTCGTGCCTG TCCCCTTTAT 360
   AGAGGATAAG TTAGCTAAAT TGGTTCTCAT TATCCTTCTG TTTTCTGGCT TTTGGACCAT 420
   CAGTGGTTTG ATC 433

### (2) ANGABEN ZU SEQ ID NO: 38:

(i) SEQUENZKENNZEICHEN:
   (A) LÄNGE: 434 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 38:
   GATCTGTATA AAGCACAAGG AGGCTTTATC TGTCTGGACT CATCTGAGGA TTCTTTGGAT 60
   CACTATTTAG ACATGTTAAA TTGATTAGAG AAGTCATTCA CTGTCATTGG GTACCATTTG 120
   TCACATCATA AACAGCTGCA GTGTGCTGCC TCCAAATGCA GAAGGACCAA CAAATAAATC 180
   ACATCAAATG GTGGGAGTAG ATGCTATCTT TCATCTATAA TTGGTATAGC CTTTCCTGTC 240
   ACATTGCTCA TTCTTATGAT TCTAGTGTGA TTGACAGACA GGGAAAGGGC AGCCTCTGTC 300
   AAAGATGTTC AGAGAGTGTG TGTTCGGGTG ATGTACCTGC TAATAGATAT CCATCATAAT 360
   ATTACATTAA TGTCACCATG CATCAAACAT ACAACTTACC CATCTAGGTA TCTGATGTTA 420
   GACCCATAAG GATC 434

### (2) ANGABEN ZU SEQ ID NO: 39:

(i) SEQUENZKENNZEICHEN:
   (A) LÄNGE: 128 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 39:
   GATCCAGCTA CAAATCTCAC AAAACACTCT GCCTGGGACA GAGGGTGTGA GTTGTAGTGG 60
   TCTGAGGCAG GAAGCTCTGA GGCAGGGCTA GAAGAATTAC CTTGGAATTA CCTTCAGAAG 120
   ACAGGATC 128

### (2) ANGABEN ZU SEQ ID NO: 40:

(i) SEQUENZKENNZEICHEN:
   (A) LANGE: 185 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 40:
   GATCAGCAAC AGATTACTAC GAAAAGGCAA GCAAACAAAC AATAACAATA ACAAAACAAA 60
   CCACTGTGTT GGATTTACTG ACTGCCTGAA ACAAATTACC CAAAGTATAG ACCGCAAAAT 120
   ATCCATGTGA ACAGGGAAAC AAAGCACACA GACAGAGCAA AAGTGGTCTC GAGGTGCTTA 180
   TGATC 185

### (2) ANGABEN ZU SEQ ID NO: 41:

(i) SEQUENZKENNZEICHEN:
   (A) LÄNGE: 129 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 41:
   GATCAAGTGA CCATAGGTGT ATGGATTCAT CTCAGGGTCT TCAATTCTGT TCCATTGGTC 60
   TACTTGTCTG TTGCCATACC AGTACCATGC ACTTTTTATC ACAAGTGCTC TGTAGTACAG 120
   CTTTAGATC 129

### (2) ANGABEN ZU SEQ ID NO: 42:

(i) SEQUENZKENNZEICHEN:
   (A) LÄNGE: 275 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 42:
   GATCTGACAC CCTCTCTGGT CTCTCTGGGC ACTGTGCTCA CGTGCTCCTC AACTCCCATN    60
   ACACGGAATT AAGAATCAAA ATATAAAATC TATAAAAAGA AAGGAAGGAA GGAAGGAAGG   120
   AAGGAAGGAA GGAAGGAAGG AAGGAAGGAA GGCAGGTAGG CAGACAGGAA GAAAGAAAGA   180
   AAGAAAGAAA GAAAGAAAGA GAGAGAGAGA GAGAGAGAGA GAGAGAAGAA GAAGAAGAAG   240
   AAGAAGAAGA AGAAGAAGAA GAAGAAGAAG AAGAA                              275

### (2) ANGABEN ZU SEQ ID NO: 43:

(i) SEQUENZKENNZEICHEN:
   (A) LÄNGE: 225 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 43:
   TCCACATTCT CCTTTGCTCT ATTATCCTTT CCTTTCCACT ACCACTGGTG AGTGATGGGC    60
   TAGAGGTTAA AGCTTATATA AACTCTTATT TAAAATAAGT TTTAAAAAAT TTAAGTCTAT   120
   AAAAGCCAGG AAAAGCAACC AATAATCAGC ATTCTTCCAT GGCCTCTGCT TCAGTTTCTG   180
   CCTCGAAGTT CCTGCCTTGA CTTCCCTCAT TAATGCAACA TGATC                   225

### (2) ANGABEN ZU SEQ ID NO: 44:

(i) SEQUENZKENNZEICHEN:
   (A) LÄNGE: 403 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 44:
   GATCTGGCCT CCCTCTTCTT GCCTTTGCAA GAACTCTATG TACATGATAT GTGTACACAT 60
   GCAAGCAAAA TACCCATGCA CACAAAATAA GGGGGTGGGA GGAACAGTCC CAGAGCGAAT 120
   GCGTGAATTA AGACAAGCCA GGTGTAGTGG CACAGCTCTG TGGTCCTAAT GCTTGGAGAA 180
   TATAATCAGG AGGATTTCTA GCTCAGTGTA TAACAAGACC TTCTCCTAAG AGAATAAAAA 240
   TAGATGTAAA TATAAATAAA TAAACATAAA ATAAGACAAT TTGTAAACTC CTTATGAGAA 300
   CTTGCTGTGG CTTTACTGGT ACATAGACCA GTAAGATTTC AGTGTAAAAG ACGCTCTCTG 360
   TAAAGGGTGA GNCAAAGGGT CCAGGAGAGG CTTTCCTTAG ATC 403

### (2) ANGABEN ZU SEQ ID NO: 45:

(i) SEQUENZKENNZEICHEN:
   (A) LÄNGE: 543 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 45:
   GATCTGAAAT TTTGAATATA TAAATAACTA ATCTTTACTA CCTTTAGATT GATGTCTTCC 60
   AAGATGAGTA TGGTTAAATA ATATTCAAAA ATCTGCATGC TAAAGATTTG TCTCAAGCAA 120
   GTCACTGTTG GGAAGTAGTT ATATTGTAAG AAGAAGTTGG GTCATAAGGA CACAGCTTGA 180
   AAGGCAAAGT AGGACTGCAC CCACTTTCTC AGCTTTTATA TTGCTTCATG ACTAGGAAAT 240
   GAAAAGTTGT CATGTGTCAC CTATCATAGC CATCCAGCAC CCAAGTCAGT GGCCAGAAGA 300
   CAGTGTGTCA GCTTGGGATG GAATGGAATC TCAAAACTGT CAATCAAATT AAACTGTGTG 360
   TGTGGAGGGC TGTATAAGTA TGTACATGTG AGTTTATGTT TGTATGTACA TAGATGCACA 420
   TGTATGTGTG TATATACATG TGTATCATGT GTGTGTGTGT GTGTGTATGT GTATGTGTGT 480
   GTGTGTGTGT GTGTGTGTGT GTGTGTGTAG TACAGAAATC AATCATGGGC ATTATTCCTG 540
   ATC 543

### (2) ANGABEN ZU SEQ ID NO: 46:

(i) SEQUENZKENNZEICHEN:
   (A) LÄNGE: 76 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 46:
   CAAGAAATGC TAGAGCTAGG GTTCCTTTTA AAATTGTCGA TGTTTCTGAT GAAAATTATT 60
   ATCAAAAGAT TAATAC 76

### (2) ANGABEN ZU SEQ ID NO: 47:

(i) SEQUENZKENNZEICHEN:
   (A) LÄNGE: 114 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 47:
   GCCTTTATTT ATTACCAAAA AGAAAAGTAT TTTCTTTAAT TTGGAAGTTT TCACAAAAGG 60
   CAATATCAAT ATTATATTGT AACATTTAAG AATTTTAAAG CCAGAAATTA AAGA 114

### (2) ANGABEN ZU SEQ ID NO: 48:

(i) SEQUENZKENNZEICHEN:
   (A) LÄNGE: 88 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 48:
   ACAGGATCAC TTGGGGACAT CTCAGGTAAC CGTGTTTTCA AAGGTATGAC ATGCCAGGTC 60
   ACTTAGGAGC TGTTAGAACA ACAGTTCA 88

### (2) ANGABEN ZU SEQ ID NO: 49:

(i) SEQUENZKENNZEICHEN:
   (A) LÄNGE: 66 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 49:
   CTTTTTAAAG TAATTAGATT TGTAACATAA GGTATTTTAA GAAAATAAAT ATTTTGATTG 60
   GTTAGT 66

### (2) ANGABEN ZU SEQ ID NO: 50:

(i) SEQUENZKENNZEICHEN:
   (A) LÄNGE: 105 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 50:
   ATGTCAACAT TTTTTTCTTG AACTAGTGCT AATAGAAGAT CAAGTTGCCT TATTTTTAAA 60
   ATAAAATTTA GTTAAAATTT TGTCAAAACC CTTTAATTTT ATTTT 105

### (2) ANGABEN ZU SEQ ID NO: 51:

(i) SEQUENZKENNZEICHEN:
   (A) LÄNGE: 52 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 51:
   CCAGATTCTC TTGCTTTTTC AGCTGTTTTA TCAGTACAAT TATGTGCTAT AA 52

(2) ANGABEN ZU SEQ ID NO: 52:
(i) SEQUENZKENNZEICHEN:
   (A) LÄNGE: 202 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 52:
   CGGACCTATT ATCCTTAATG AATTACTTAA AGTTGCTGAC ACTCGTTCAG TTCAAAACTA 60
   CTTATTGAAA GAAGTACAAA GACTTTATCG TCTTCAAGGT ATCACAATTA GTGATAAATA 120
   TATTGAAATG ATTATTCGTC AAATGCTTTC AAAAATTGTT ATTACTGATC CAGGAGATAG 180
   TAAATTCTTT AGTGGTAACT TA 202

### (2) ANGABEN ZU SEQ ID NO: 53:

(i) SEQUENZKENNZEICHEN:
   (A) LÄNGE: 170 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 53:
   AAGAAGATTA ACTTACAAAG ATTTTTTGGA AAAATATAAA CTTATTATGG ATAAAAATAC 60
   TATTCTAAAA TTCAAATCAG ATAATGATAA ACTTTATGAA TTTTCATTAG AAAGCTTTAA 120
   AGAAAACGAT AAATATAATT TCATATGGCA GAGATTTGCA TAAAAGCAAA 170

### (2) ANGABEN ZU SEQ ID NO: 54:

(i) SEQUENZKENNZEICHEN:
   (A) LÄNGE: 188 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 54:
   TTGAGTTTTA TTAGTTGAAG TTTTAAAGCG CTAGTACAAG AAAATTTTTC AGAAAACGTT 60
   CTTATTGTAC TTTGAACGTG GTTGTAGAAA TTTAAAAAAC ACACTAGTTA TTTGAGGATT 120
   CTGTAGTGAA GGCGGACATG AACTCAATTA ATAATAATTA TTTCGGCGAC GATAAGGACG 180
   AACATTTT 188

### (2) ANGABEN ZU SEQ ID NO: 55:

(i) SEQUENZKENNZEICHEN:
   (A) LÄNGE: 230 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 55:
   CTTTAACAAT TCTTACATTT AAAACTTCTA CTAATACATC TAAATGTTTT TTAACTCCAT 60
   GGCTCGAGCT TAAGCATTAG TACCAGTATC GACAAAGGCA CACATTTAAC AATAGGCGAG 120
   TGTTAAGGTG TGTTGTATGC TCGGCCTTCG TATTTCACAT TCGGACCCCA CGGATTACTC 180
   ACTCGATTGA GTGTAATTAA CGCAACGCGA GTGACGGCGA AGTCAGCCTT 230

### (2) ANGABEN ZU SEQ ID NO: 56:

(i) SEQUENZKENNZEICHEN:
   (A) LÄNGE: 139 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 56:
   CACTTGTAGG TACTACAGTT GTAAAAAAAG AACTTGATCA CGATTATCTT CTAGTTCAAC 60
   GGAATAAAAA TTTTATTTTA AATCAATTTT AAAACAGTTT TGGAAATTAG AAAATAAAAC 120
   CCATGGCTCG AGCTTAGCA 139

### (2) ANGABEN ZU SEQ ID NO: 57:

(i) SEQUENZKENNZEICHEN:
   (A) LÄNGE: 225 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 57:
   ACGTCCGTAC GTTCGAACCG CATTACTACC AGTATCGACA AAGGCACACA TTTAACAATA 60
   GGCGATTGTT AAGGTGTGTT GTAAGCTCGG CCTTCGTATT TCACATTTCG GACCCCACGG 120
   ATTACTCACT CGATTGAGTG TAATTAACGC AACGCGAGTG ACGGGCGAAA GGTCAGCCCT 180
   TTGGACAGCA CGGTCGACGT AATTACTTAG CCGGTTGCGC GCCCC 225

### (2) ANGABEN ZU SEQ ID NO: 58:

(i) SEQUENZKENNZEICHEN:
   (A) LÄNGE: 22 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 58:
   ACCTGCAGGC ATGCAAGCTT GG 22

### (2) ANGABEN ZU SEQ ID NO: 59:

(i) SEQUENZKENNZEICHEN:
   (A) LÄNGE: 215 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 59:
   CCCTAAAATT AAAGAATTTA AAGAATTAGT TAAAAGCAGC AAAGAATTAT TCTTACAAAA    60
   AATACAAGCA AAATTTGAAA TGACTAAAAC GAGATTCAAG AATCAAGCCT TGCATTGTTT   120
   CAATAATCAA TTAGCAACTT TTTCAATAGT AAAAGAAAAA GTTATTCAAC GTAATCCATT   180
   AAAATATTAG AAAGTTTCGA AGTTACAATG AACAC                              215

### (2) ANGABEN ZU SEQ ID NO: 60:

(i) SEQUENZKENNZEICHEN:
   (A) LÄNGE: 192 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 60:
   TAATTGAAAA ACATGTTGAT GAAGTCGATA TGGTAGAAAA TTAAGTTACA TTTCAAAAAA    60
   TAGTTGAAAT TGAACAAAAT AACAAAATTA ATAAACCAAC AAAAGTAAAC ATTGAAAACG   120
   TTTTTGAAAA AGATTATAAA AATTACCTAA TGTAACTTAT TGAAAAAGAA AATGATAACT   180
   TTTTCAACGA TT                                                       192

### (2) ANGABEN ZU SEQ ID NO: 61:

(i) SEQUENZKENNZEICHEN:
   (A) LÄNGE: 91 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 61:
   ATGACCAAAA TAAATTAACG TGAGTTTTCG TTCCACTGAG CGTCAGACCC CGTAGAAAAG 60
   ATCAAAGGAT GTTCTTGAGA CCTTTTTTTC T 91

### (2) ANGABEN ZU SEQ ID NO: 62:

(i) SEQUENZKENNZEICHEN:
   (A) LÄNGE: 27 Basenpaare
   (B) ART: Nucleotid
   (C) STRANGFORM: Einzelstrang
   (D) TOPOLOGIE: linear
(ii) ART DES MOLEKÜLS: Genom-DNA
(xi) SEQUENZBESCHREIBUNG: SEQ ID NO: 62:
   NNAAANTNTN GAANTGTANN ANTGNAA 27